# EUROPEAN PATENT SPECIFICATION

(11) **EP 4 204 052 B1**
(45) Date of publication and mention of the grant of the patent: **25.03.2026**
(21) Application number: 21777911.5
(22) Date of filing: 30.08.2021
(51) Int. Cl.: A61M 16/00, A61B 5/085, A61B 5/00, A61B 8/08, A61M 11/02, A61M 11/06, A61M 16/06, A61M 16/10

(54) **SYSTEMS FOR PRE-SYMPTOMATIC DISEASE DETECTION**
SYSTEME FÜR DEN NACHWEIS PRÄSYMPTOMATISCHER ERKRANKUNGEN
SYSTÈMES DE DÉTECTION DE MALADIES PRÉ-SYMPTOMATIQUES

(30) Priority: 31.08.2020 US 202063072896 P
(43) Date of publication of application: 05.07.2023
(73) Proprietor: ResMed Sensor Technologies Limited, Sandyford D18 T6T7 Dublin (IE)
(72) Inventor: DOS SANTOS, Jose, Ricardo, San Diego, California 92123 (US); SHOULDICE, Redmond, Sandyford D18 T6T7 Dublin (IE)
(74) Representative: Mathys & Squire
(86) International application number: PCT/US2021/048295
(87) International publication number: WO 2022/047339

(56) References cited:
- WO-A1-03/024335
- WO-A1-2010/091462
- WO-A2-2020/092701
- AU-B2- 2013 201 312

## Description

### CROSS-REFERENCE TO RELATED APPLICATIONS

This application claims the benefit of, and priority to, U.S. Provisional Patent Application No. 63/072,896 filed on August 31, 2020.

### TECHNICAL FIELD

The present disclosure relates generally to systems and methods for monitoring an individual's airway, and more particularly, to systems and methods for detecting and identifying respiratory conditions prior to physical symptoms by monitoring the individual's airway.

### BACKGROUND

Many individuals suffer from sleep-related and/or respiratory disorders such as, for example, Periodic Limb Movement Disorder (PLMD), Restless Leg Syndrome (RLS), Sleep-Disordered Breathing (SDB), such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders. These individuals can also suffer from other respiratory conditions. Often times, respiratory conditions are not able to be detected and identified until the individual presents with externally-detectable physical symptoms, such as a cough, stuffiness, fever, etc. Thus, it would be advantageous to be able to accurately monitor the individual's airway to be able to detect and identify potential respiratory conditions before those respiratory conditions result in externally-detectable physical symptoms. Documents WO2010/091462 and AU2013201312 disclose technologies implementing obstruction detection, component or accessory detection and/or patient or user detection by acoustic analysis. Document WO03/024335 concerns the determination of apnoea and/or hypopnoea in a patient by applying variable frequency ultrasonic sound waves to the patient's airways and analysing the reflected sound waves to determine whether there is a narrowing of the airways.

The present disclosure is directed to systems, devices, and methods to allow for easier monitoring of an individual's airway.

### SUMMARY

The invention is defined by the appended claims. Subject-matter referred as embodiments, disclosures and/or implementations which does not fall under the scope of the claims is not part of the invention.

According to some implementations of the present disclosure, a method (not claimed) of monitoring an airway of an individual comprises causing an acoustic signal to be directed into the airway of the individual. The method further comprises generating acoustic data representative of one or more reflections of the acoustic signal. The reflections are caused by (i) a portion of the airway of the individual, (ii) an obstruction within the airway of the individual, or (iii) both (i) and (ii). The method further comprises analyzing the acoustic data to determine a value of a parameter associated with the airway of the individual. The method further comprises causing an action to be performed, based on the determined value of the parameter.

According to some implementations of the present disclosure, a system comprises a respiratory therapy system, a memory storing machine-readable instructions, and a control system. The respiratory therapy system includes a respiratory therapy device and a user interface. The respiratory therapy device is configured to supply pressurized air. The user interface is coupled to the respiratory therapy device via a conduit, and is configured to engage the individual and aid in directing the supplied pressurized air to an airway of the individual. The control system includes one or more processors configured to execute the machine-readable instructions to: cause an acoustic signal to be directed, via the conduit and the user interface, into the airway of the individual; generate acoustic data representative of one or more reflections of the acoustic signal caused by (i) a portion of the airway of the individual, (ii) an obstruction within the airway of the individual, or (iii) both (i) and (ii); analyze the acoustic data to determine a value of a parameter associated with the airway of the individual; and based on the determined value of the parameter, cause an action to be performed

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system for monitoring a sleep session, according to some implementations of the present disclosure;
FIG. 2 is a perspective view of the system of FIG. 1, a user of the system, and a bed partner of the user, according to some implementations of the present disclosure;
FIG. 3 illustrates an exemplary timeline for a sleep session, according to some implementations of the present disclosure;
FIG. 4 illustrates an exemplary hypnogram associated with the sleep session of FIG. 3, according to some implementations of the present disclosure;
FIG. 5 is an overview of a respiratory therapy system of a user, according to some implementations of the present disclosure;
FIG. 6A is an exemplary cepstrum plot representing a physical obstruction in an airway of a user, according to some implementations of the present disclosure;
FIG. 6B is a cross-sectional view of a user interface with a microphone and a speaker being used to characterize the physical obstruction in the airway of the user, according to some implementations of the present disclosure;
FIG. 7A is an exemplary cepstrum plot representing the structure of the airway of the user, according to some implementations of the present disclosure;
FIG. 7B is a cross-sectional view of a user interface with a microphone and a speaker being used to characterize the airway of the user, according to some implementations of the present disclosure; and
FIG. 8 is a process flow diagram for a method of monitoring the airway of the user, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the spirit and scope of the present disclosure as defined by the appended claims.

### DETAILED DESCRIPTION

The present disclosure is described with reference to the attached figures, where like reference numerals are used throughout the figures to designate similar or equivalent elements. The figures are not drawn to scale, and are provided merely to illustrate the instant disclosure. Several aspects of the disclosure are described below with reference to example applications for illustration.

Many individuals suffer from sleep-related and/or respiratory-related disorders such as, for example, Periodic Limb Movement Disorder (PLMD), Restless Leg Syndrome (RLS), Sleep-Disordered Breathing (SDB) such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hyper tension, diabetes, stroke, insomnia, and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall.

Central Sleep Apnea (CSA) is another form of SDB that results when the brain temporarily stops sending signals to the muscles that control breathing. More generally, an apnea generally refers to the cessation of breathing caused by blockage of the air or the stopping of the breathing function. Typically, the individual will stop breathing for between about 15 seconds and about 30 seconds during an obstructive sleep apnea event. Mixed sleep apnea is another form of SDB that is a combination of OSA and CSA.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

A Respiratory Effort Related Arousal (RERA) event is typically characterized by an increased respiratory effort for 10 seconds or longer leading to arousal from sleep and which does not fulfill the criteria for an apnea or hypopnea event. In 1999, the AASM Task Force defined RERAs as "a sequence of breaths characterized by increasing respiratory effort leading to an arousal from sleep, but which does not meet criteria for an apnea or hypopnea. These events must fulfil both of the following criteria: 1. pattern of progressively more negative esophageal pressure, terminated by a sudden change in pressure to a less negative level and an arousal; 2. the event lasts 10 seconds or longer. In 2000, the study "Non-Invasive Detection of Respiratory Effort-Related Arousals (RERAs) by a Nasal Cannula/Pressure Transducer System" done at NYU School of Medicine and published in Sleep, vol. 23, No. 6, pp. 763-771, demonstrated that a Nasal Cannula/Pressure Transducer System was adequate and reliable in the detection of RERAs. A RERA detector may be based on a real flow signal derived from a respiratory therapy (e,g, PAP) device. For example, a flow limitation measure may be determined based on a flow signal. A measure of arousal may then be derived as a function of the flow limitation measure and a measure of sudden increase in ventilation. One such method is described in WO 2008/138040, US 9,358,353, US 10,549,053, and US 2020/0197640.

Cheyne-Stokes Respiration (CSR) is another form of SDB. CSR is a disorder of a patient's respiratory controller in which there are rhythmic alternating periods of waxing and waning ventilation known as CSR cycles. CSR is characterized by repetitive de-oxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

Individuals with many of these disorders can also suffer from other respiratory conditions, such as lung disease, lung irritation, lung inflammation, etc. However, such respiratory conditions are often not able to be detected and identified until the individual presents with externally-detectable physical symptoms, such as a cough, stuffiness, fever, etc. Thus, it would be advantageous to be able to accurately monitor the individual's airway to be able to detect and/or identify potential respiratory conditions before those respiratory conditions result in externally-detectable physical symptoms.

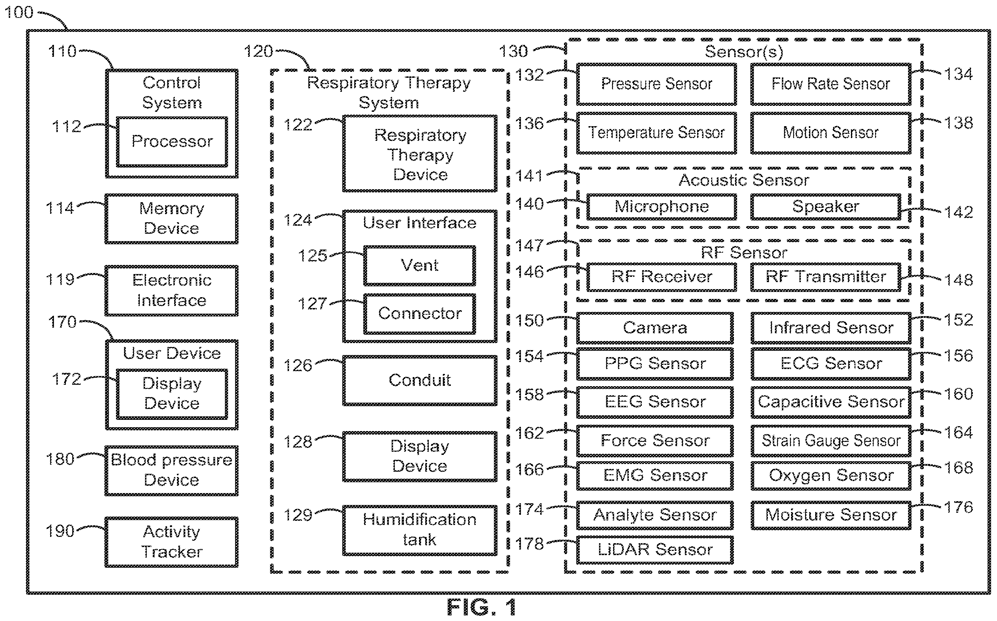

Referring to FIG. 1, a system 100, according to some implementations of the present disclosure, is illustrated. The system 100 can be used detect and/or identify respiratory conditions before the manifestations of external physical symptoms. The system 100 includes a control system 110, a memory device 114, an electronic interface 119, one or more sensors 130, and optionally one or more user devices 170. In some implementation, the system 100 further includes a respiratory therapy system 120 that includes a respiratory therapy device 122.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the user device 170, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122 of the respiratory therapy system 120, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family medical history (such as a family history of insomnia or sleep apnea), an employment status of the user, an educational status of the user, a socioeconomic status of the user, or any combination thereof. The medical information can include, for example, information indicative of one or more medical conditions associated with the user, medication usage by the user, or both. The medical information data can further include a fall risk assessment associated with the user (e.g., a fall risk score using the Morse fall scale), a multiple sleep latency test (MSLT) result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or acoustic data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, an IR communication protocol, over a cellular network, over any other optical communication protocol, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 optionally includes a respiratory therapy system 120 (also referred to as a respiratory pressure therapy system). The respiratory therapy system 120 can include a respiratory therapy device 122 (also referred to as a respiratory pressure device), a user interface 124 (also referred to as a mask or a patient interface), a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea), other respiratory disorders such as COPD, or other disorders leading to respiratory insufficiency, that may manifest either during sleep or wakefulness.

The respiratory therapy device 122 is generally used to generate pressurized air that is delivered to a user (e.g., using one or more motors (such as a blower motor) that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure). In some implementations, the control system 110, the memory device 114, the electronic interface 119, or any combination thereof can be coupled to and/or positioned within a housing of the respiratory therapy device 122.

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cmH₂O.

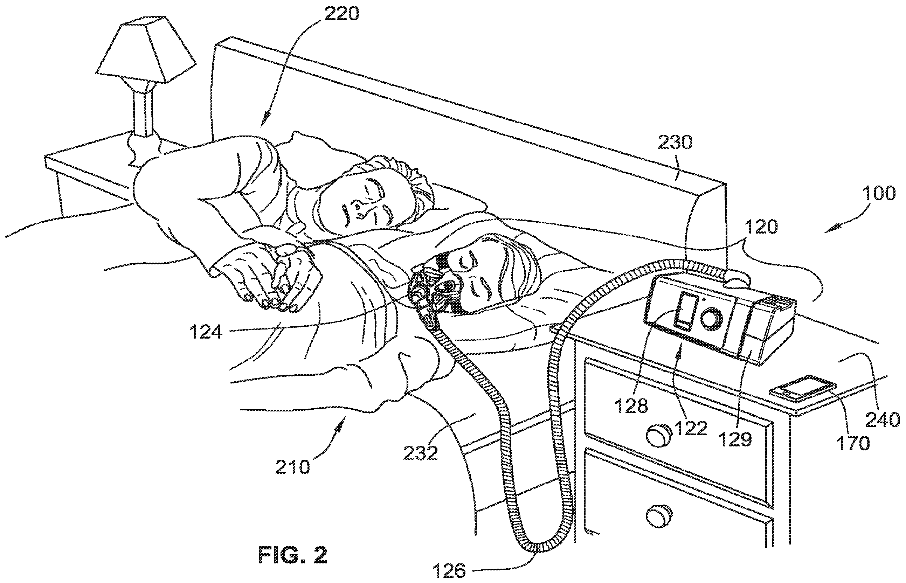

In some implementations, the user interface 124 is or includes a facial mask that covers the nose and mouth of the user (as shown, for example, in FIG. 2). Alternatively, the user interface 124 is or includes a nasal mask that provides air to the nose of the user or a nasal pillow mask that delivers air directly to the nostrils of the user. The user interface 124 can include a strap assembly that has a plurality of straps (e.g., including hook and loop fasteners) for positioning and/or stabilizing the user interface 124 on a portion of the user interface 124 on a desired location of the user (e.g., the face), and a conformal cushion (e.g., silicone, plastic, foam, etc.) that aids in providing an air-tight seal between the user interface 124 and the user. In some implementations, the user interface 124 may include a connector 127 and one or more vents 125. The one or more vents 125 can be used to permit the escape of carbon dioxide and other gases exhaled by the user. In other implementations, the user interface 124 includes a mouthpiece (e.g., a night guard mouthpiece molded to conform to the user's teeth, a mandibular repositioning device, etc.). In some implementations, the connector 127 is distinct from, but couplable to, the user interface 124 (and/or conduit 126). The connector 127 is configured to connect and fluidly couple the user interface 124 to the conduit 126.

The conduit 126 allows the flow of air between two components of a respiratory therapy system 120, such as the respiratory therapy device 122 and the user interface 124. In some implementations, there can be separate limbs of the conduit for inhalation and exhalation. In other implementations, a single limb conduit is used for both inhalation and exhalation. Generally, the respiratory therapy system 120 forms an air pathway that extends between a motor of the respiratory therapy device 122 and the user and/or the user's airway. Thus, the air pathway generally includes at least a motor of the respiratory therapy device 122, the user interface 124, and the conduit 126.

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score or a therapy score (such as a myAir^{™} score, such as described in WO 2016/061629 and US 2017/0311879), the current date/time, personal information for the user, a questionnaire for the user, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir of water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself. In other implementations, the respiratory therapy device 122 or the conduit 126 can include a waterless humidifier. The waterless humidifier can incorporate sensors that interface with other sensor positioned elsewhere in system 100.

The respiratory therapy system 120 can be used, for example, as a ventilator or a positive airway pressure (PAP) system, such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based at least in part on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can include the display device 128, which can allow the user to interact with the respiratory therapy device 122. The respiratory therapy device 122 can also include the humidification tank 129, which stores the water used to humidify the pressurized air. The respiratory therapy device 122 can be positioned on a nightstand 240 that is directly adjacent to the bed 230 as shown in FIG. 2, or more generally, on any surface or structure that is generally adjacent to the bed 230 and/or the user 210. The user can also wear the blood pressure device 180 and the activity tracker 190 while lying on the mattress 232 in the bed 230.

Referring back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, an RF transmitter 148, a camera 150, an infrared (IR) sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a light detection and ranging (LiDAR) sensor 178, or any combination thereof. Generally, each of the one or sensors 130 are configured to output sensor data that is received and stored in the memory device 114 or one or more other memory devices. The sensors 130 can also include, an electrooculography (EOG) sensor, a peripheral oxygen saturation (SpO₂) sensor, a galvanic skin response (GSR) sensor, a carbon dioxide (CO₂) sensor, or any combination thereof.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the IR sensor 152, the PPG sensor 154, the ECG sensor 156, the EEG sensor 158, the capacitive sensor 160, the force sensor 162, the strain gauge sensor 164, the EMG sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example physiological data, acoustic data, or both, that is associated with a user of the respiratory therapy system 120 (such as the user 210 of FIG. 2), the respiratory therapy system 120, both the user and the respiratory therapy system 120, or other entities, objects, activities, etc. Physiological data generated by one or more of the sensors 130 can be used by the control system 110 to determine a sleep-wake signal associated with the user during the sleep session and one or more sleep-related parameters. The sleep-wake signal can be indicative of one or more sleep stages (sometimes referred to as sleep states), including sleep, wakefulness, relaxed wakefulness, micro-awakenings, or distinct sleep stages such as a rapid eye movement (REM) stage (which can include both a typical REM stage and an atypical REM stage), a first non-REM stage (often referred to as "N1"), a second non-REM stage (often referred to as "N2"), a third non-REM stage (often referred to as "N3"), or any combination thereof. Methods for determining sleep stages from physiological data generated by one or more of the sensors, such as sensors 130, are described in, for example, WO 2014/047310, US 10,492,720, US 10,660,563, US 2020/0337634, WO 2017/132726, WO 2019/122413, US 2021/0150873, WO 2019/122414, US 2020/0383580.

The sleep-wake signal can also be timestamped to indicate a time that the user enters the bed, a time that the user exits the bed, a time that the user attempts to fall asleep, etc. The sleep-wake signal can be measured one or more of the sensors 130 during the sleep session at a predetermined sampling rate, such as, for example, one sample per second, one sample per 30 seconds, one sample per minute, etc. Examples of the one or more sleep-related parameters that can be determined for the user during the sleep session based at least in part on the sleep-wake signal include a total time in bed, a total sleep time, a total wake time, a sleep onset latency, a wake-after-sleep-onset parameter, a sleep efficiency, a fragmentation index, an amount of time to fall asleep, a consistency of breathing rate, a fall asleep time, a wake time, a rate of sleep disturbances, a number of movements, or any combination thereof.

Physiological data and/or acoustic data generated by the one or more sensors 130 can also be used to determine a respiration signal associated with the user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration amplitude ratio, an inspiration-expiration duration ratio, a number of events per hour, a pattern of events, pressure settings of the respiratory therapy device 122, or any combination thereof. The event(s) can include snoring, apneas, central apneas, obstructive apneas, mixed apneas, hypopneas, RERAs, a flow limitation (e.g., an event that results in the absence of the increase in flow despite an elevation in negative intrathoracic pressure indicating increased effort), a mask leak (e.g., from the user interface 124), a restless leg, a sleeping disorder, choking, an increased heart rate, a heart rate variation, labored breathing, an asthma attack, an epileptic episode, a seizure, a fever, a cough, a sneeze, a snore, a gasp, the presence of an illness such as the common cold or the flu, an elevated stress level, etc. Events can be detected by any means known in the art such as described in, for example, US 5,245,995, US 6,502,572, WO 2018/050913, WO 2020/104465.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, an inductive sensor, a resistive sensor, a piezoelectric sensor, a strain-gauge sensor, an optical sensor, a potentiometric sensor, or any combination thereof. In one example, the pressure sensor 132 can be used to determine a blood pressure of the user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user, a skin temperature of the user 210, a temperature of the air flowing from the respiratory therapy device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. The motion sensor 138 can be used to detect motion or acceleration associated with arterial pulses, such as pulses in or around the face of the user and proximal to the user interface 124, and configured to detect features of the pulse shape, speed, amplitude, or volume. In some implementations, the motion sensor 138 alternatively or additionally generates one or more signals representing bodily movement of the user, from which may be obtained a signal representing a sleep state of the user; for example, via a respiratory movement of the user.

The microphone 140 outputs acoustic data that can be stored in the memory device 114 and/or analyzed by the processor 112 of the control system 110. The acoustic data generated by the microphone 140 is reproducible as one or more sound(s) during a sleep session (e.g., sounds from the user) to determine (e.g., using the control system 110) one or more sleep-related parameters, as described in further detail herein. The acoustic data from the microphone 140 can also be used to identify (e.g., using the control system 110) an event experienced by the user during the sleep session, as described in further detail herein. In other implementations, the acoustic data from the microphone 140 is representative of noise associated with the respiratory therapy system 120. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones. The microphone 140 can be coupled to or integrated in the respiratory therapy system 120 (or the system 100) generally in any configuration. For example, the microphone 140 can be disposed inside the respiratory therapy device 122, the user interface 124, the conduit 126, or other components. The microphone 140 can also be positioned adjacent to or coupled to the outside of the respiratory therapy device 122, the outside of the user interface 124, the outside of the conduit 126, or outside of any other components. The microphone 140 could also be a component of the user device 170 (e.g., the microphone 140 is a microphone of a smart phone). The microphone 140 can be integrated into the user interface 124, the conduit 126, the respiratory therapy device 122, or any combination thereof. In general, the microphone 140 can be located at any point within or adjacent to the air pathway of the respiratory therapy system 120, which includes at least the motor of the respiratory therapy device 122, the user interface 124, and the conduit 126. Thus, the air pathway can also be referred to as the acoustic pathway.

The speaker 142 outputs sound waves that are audible to the user. In one or more implementations, the sound waves can be audible to a user of the system 100 or inaudible to the user of the system (e.g., ultrasonic sound waves). The speaker 142 can be used, for example, as an alarm clock or to play an alert or message to the user (e.g., in response to an event). In some implementations, the speaker 142 can be used to communicate the acoustic data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and/or frequency, and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user or a bed partner of the user (such as bed partner 220 in FIG. 2). Based at least in part on the data from the microphone 140 and/or the speaker 142, the control system 110 can determine a location of the user and/or one or more of the sleep-related parameters described in herein, such as, for example, a respiration signal, a respiration rate, an inspiration amplitude, an expiration amplitude, an inspiration-expiration ratio, a number of events per hour, a pattern of events, a sleep stage, pressure settings of the respiratory therapy device 122, a mouth leak status, or any combination thereof. In this context, a SONAR sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO 2018/050913 and WO 2020/104465 mentioned above. In some implementations, the speaker 142 is a bone conduction speaker. In some implementations, the one or more sensors 130 include (i) a first microphone that is the same or similar to the microphone 140, and is integrated into the acoustic sensor 141 and (ii) a second microphone that is the same as or similar to the microphone 140, but is separate and distinct from the first microphone that is integrated into the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor). In some such implementations, the RF sensor 147 includes a control circuit. The specific format of the RF communication could be WiFi, Bluetooth, etc.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based at least in part on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user enters the user's bed (such as bed 230 in FIG. 2), and to determine a time when the user exits the bed 230. The camera 150 can also be used to track eye movements, pupil dilation (if one or both of the user's eyes are open), blink rate, or any changes during REM sleep. The camera 150 can also be used to track the position of the user, which can impact the duration and/or severity of apneic episodes in users with positional obstructive sleep apnea.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user that can be used to determine one or more sleep-related parameters, such as, for example, a heart rate, a heart rate pattern, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user, embedded in clothing and/or fabric that is worn by the user, embedded in and/or coupled to the user interface 124 and/or its associated headgear (e.g., straps, etc.), etc.

The ECG sensor 156 outputs physiological data associated with electrical activity of the heart of the user. In some implementations, the ECG sensor 156 includes one or more electrodes that are positioned on or around a portion of the user during the sleep session. The physiological data from the ECG sensor 156 can be used, for example, to determine one or more of the sleep-related parameters described herein.

The EEG sensor 158 outputs physiological data associated with electrical activity of the brain of the user. In some implementations, the EEG sensor 158 includes one or more electrodes that are positioned on or around the scalp of the user during the sleep session. The physiological data from the EEG sensor 158 can be used, for example, to determine a sleep stage of the user at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user. The data output by the analyte sensor 174 can be stored in the memory device 114 and used by the control system 110 to determine the identity and concentration of any analytes in the user's breath. In some implementations, the analyte sensor 174 is positioned near a mouth of the user to detect analytes in breath exhaled from the user's mouth. For example, when the user interface 124 is a facial mask that covers the nose and mouth of the user, the analyte sensor 174 can be positioned within the facial mask to monitor the user mouth breathing. In other implementations, such as when the user interface 124 is a nasal mask or a nasal pillow mask, the analyte sensor 174 can be positioned near the nose of the user to detect analytes in breath exhaled through the user's nose. In still other implementations, the analyte sensor 174 can be positioned near the user's mouth when the user interface 124 is a nasal mask or a nasal pillow mask. In this implementation, the analyte sensor 174 can be used to detect whether any air is inadvertently leaking from the user's mouth. In some implementations, the analyte sensor 174 is a volatile organic compound (VOC) sensor that can be used to detect carbon-based chemicals or compounds, such as carbon dioxide. In some implementations, the analyte sensor 174 can also be used to detect whether the user is breathing through their nose or mouth. For example, if the data output by an analyte sensor 174 positioned near the mouth of the user or within the facial mask (in implementations where the user interface 124 is a facial mask) detects the presence of an analyte, the control system 110 can use this data as an indication that the user is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user's face, near the connection between the conduit 126 and the user interface 124, near the connection between the conduit 126 and the respiratory therapy device 122, etc.). Thus, in some implementations, the moisture sensor 176 can be coupled to or integrated into the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user, for example the air inside the user's bedroom. The moisture sensor 176 can also be used to track the user's biometric response to environmental changes.

One or more LiDAR sensors 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor 178 may also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170, or any combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, the pressure sensor 132 and/or the flow rate sensor 134 are integrated into and/or coupled to the respiratory therapy device 122. In some implementations, at least one of the one or more sensors 130 is not coupled to the respiratory therapy device 122, the control system 110, or the user device 170, and is positioned generally adjacent to the user during the sleep session (e.g., positioned on or in contact with a portion of the user, worn by the user, coupled to or positioned on the nightstand, coupled to the mattress, coupled to the ceiling, etc.). More generally, the one or more sensors 130 can be positioned at any suitable location relative to the user such that the one or more sensors 130 can generate physiological data associated with the user and/or the bed partner 220 during one or more sleep session.

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, an average duration of events, a range of event durations, a ratio between the number of different events, a sleep stage, an apnea-hypopnea index (AHI), or any combination thereof. The one or more events can include snoring, apneas, central apneas, obstructive apneas, mixed apneas, hypopneas, an intentional user interface leak, an unintentional user interface leak, a mouth leak, a cough, a restless leg, a sleeping disorder, choking, an increased heart rate, labored breathing, an asthma attack, an epileptic episode, a seizure, increased blood pressure, hyperventilation, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 includes a display device 172. The user device 170 can be, for example, a mobile device such as a smart phone, a tablet, a laptop, a gaming console, a smart watch, or the like. Alternatively, the user device 170 can be an external sensing system, a television (e.g., a smart television) or another smart home device (e.g., a smart speaker(s) such as Google Home, Amazon Echo, Alexa etc.). In some implementations, the user device 170 is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices 170 can be used by and/or included in the system 100.

The blood pressure device 180 is generally used to aid in generating physiological data for determining one or more blood pressure measurements associated with a user. The blood pressure device 180 can include at least one of the one or more sensors 130 to measure, for example, a systolic blood pressure component and/or a diastolic blood pressure component.

In some implementations, the blood pressure device 180 is a sphygmomanometer including an inflatable cuff that can be worn by a user and a pressure sensor (e.g., the pressure sensor 132 described herein). For example, as shown in the example of FIG. 2, the blood pressure device 180 can be worn on an upper arm of the user. In such implementations where the blood pressure device 180 is a sphygmomanometer, the blood pressure device 180 also includes a pump (e.g., a manually operated bulb) for inflating the cuff. In some implementations, the blood pressure device 180 is coupled to the respiratory therapy device 122 of the respiratory therapy system 120, which in turn delivers pressurized air to inflate the cuff. More generally, the blood pressure device 180 can be communicatively coupled with, and/or physically integrated in (e.g., within a housing), the control system 110, the memory device 114, the respiratory therapy system 120, the user device 170, and/or the activity tracker 190.

The activity tracker 190 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum respiration rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), or any combination thereof. The activity tracker 190 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 190 is a wearable device that can be worn by the user, such as a smartwatch, a wristband, a ring, or a patch. For example, referring to FIG. 2, the activity tracker 190 is worn on a wrist of the user. The activity tracker 190 can also be coupled to or integrated a garment or clothing that is worn by the user. Alternatively, still, the activity tracker 190 can also be coupled to or integrated in (e.g., within the same housing) the user device 170. More generally, the activity tracker 190 can be communicatively coupled with, or physically integrated in (e.g., within a housing), the control system 110, the memory device 114, the respiratory therapy system 120, the user device 170, and/or the blood pressure device 180.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for identifying a user interface used by a user, according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. As a further example, a fourth alternative system includes the control system 110, the memory device 114, the respiratory therapy system 120, at least one of the one or more sensors 130, the user device 170, and the blood pressure device 180 and/or activity tracker 190. Thus, various systems for modifying pressure settings can be formed using any portion or portions of the components shown and described herein and/or in combination with one or more other components.

Referring again to FIG. 2, in some implementations, the control system 110, the memory device 114, any of the one or more sensors 130, or a combination thereof can be located on and/or in any surface and/or structure that is generally adjacent to the bed 230 and/or the user 210. For example, in some implementations, at least one of the one or more sensors 130 can be located at a first position on and/or in one or more components of the respiratory therapy system 120 adjacent to the bed 230 and/or the user 210. The one or more sensors 130 can be coupled to the respiratory therapy system 120, the user interface 124, the conduit 126, the display device 128, the humidification tank 129, or a combination thereof.

Alternatively, or additionally, at least one of the one or more sensors 130 can be located at a second position on and/or in the bed 230 (e.g., the one or more sensors 130 are coupled to and/or integrated in the bed 230). Further, alternatively or additionally, at least one of the one or more sensors 130 can be located at a third position on and/or in the mattress 232 that is adjacent to the bed 230 and/or the user 210 (e.g., the one or more sensors 130 are coupled to and/or integrated in the mattress 232). Alternatively, or additionally, at least one of the one or more sensors 130 can be located at a fourth position on and/or in a pillow that is generally adjacent to the bed 230 and/or the user 210.

Alternatively, or additionally, at least one of the one or more sensors 130 can be located at a fifth position on and/or in the nightstand 240 that is generally adjacent to the bed 230 and/or the user 210. Alternatively, or additionally, at least one of the one or more sensors 130 can be located at a sixth position such that the at least one of the one or more sensors 130 are coupled to and/or positioned on the user 210 (e.g., the one or more sensors 130 are embedded in or coupled to fabric, clothing, and/or a smart device worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. For example, one or more microphones (the same as, or similar to, the microphone 140 of FIG. 1) can be integrated in and/or coupled to (i) a circuit board of the respiratory therapy device 122, (ii) the conduit 126, (iii) a connector between components of the respiratory therapy system 120, (iv) the user interface 124, (v) a headgear (e.g., straps) associated with the user interface, or (vi) a combination thereof.

In some implementations, one or more secondary sensors may be used in addition to the primary sensor to generate additional data. In some such implementations, the one or more secondary sensors include: a microphone (e.g., the microphone 140 of the system 100), a flow rate sensor (e.g., the flow rate sensor 134 of the system 100), a pressure sensor (e.g., the pressure sensor 132 of the system 100), a temperature sensor (e.g., the temperature sensor 136 of the system 100), a camera (e.g., the camera 150 of the system 100), a vane sensor (VAF), a hot wire sensor (MAF), a cold wire sensor, a laminar flow sensor, an ultrasonic sensor, an inertial sensor, or a combination thereof.

Additionally, or alternatively, one or more microphones (the same as, or similar to, the microphone 140 of FIG. 1) can be integrated in and/or coupled to a co-located smart device, such as the user device 170, a TV, a watch (e.g., a mechanical watch or another smart device worn by the user), a pendant, the mattress 232, the bed 230, beddings positioned on the bed 230, the pillow, a speaker (e.g., the speaker 142 of FIG. 1), a radio, a tablet device, a waterless humidifier, or a combination thereof. A co-located smart device can be any smart device that is within range for detecting sounds emitted by the user, the respiratory therapy system 120, and/or any portion of the system 100. In some implementations, the co-located smart device is a smart device that is in the same room as the user during the sleep session.

Additionally, or alternatively, in some implementations, one or more microphones (the same as, or similar to, the microphone 140 of FIG. 1) can be remote from the system 100 (FIG. 1) and/or the user 210 (FIG. 2), so long as there is an air passage allowing acoustic signals to travel to the one or more microphones. For example, the one or more microphones can be in a different room from the room containing the system 100.

As used herein, a sleep session can be defined in a number of ways based at least in part on, for example, an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

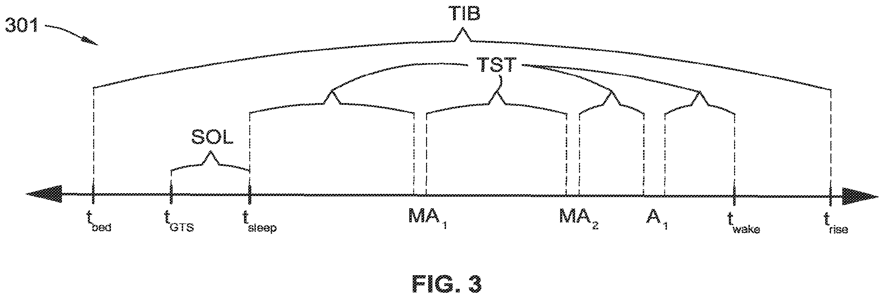

Referring to FIG. 3, an exemplary timeline 301 for a sleep session is illustrated. The timeline 301 includes an enter bed time (t_{bed}), a go-to-sleep time (t_{GTS}), an initial sleep time (tₛₗₑₑₚ), a first micro-awakening MA₁, a second micro-awakening MA₂, an awakening A, a wake-up time (t_{wake}), and a rising time (tᵣᵢₛₑ).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based at least in part on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MAᵢ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based at least in part on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based at least in part on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based at least in part on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based at least in part on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (t_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based at least in part on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 301 of FIG. 3, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (t_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

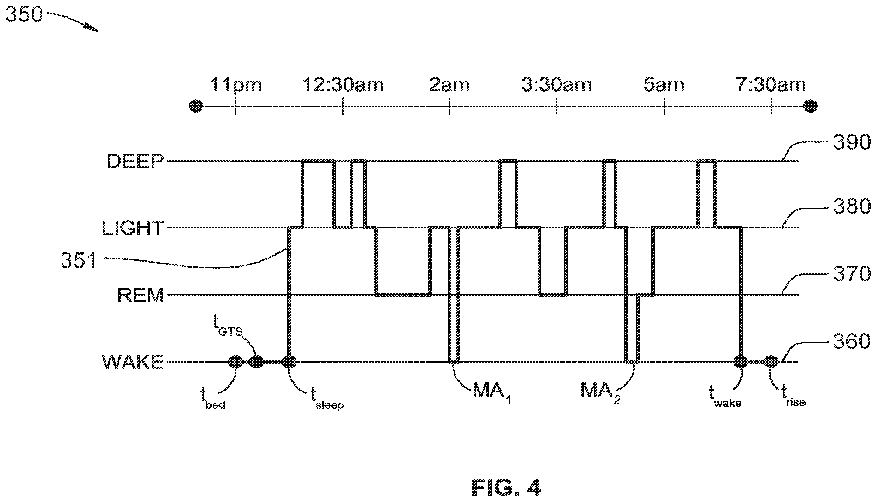

Referring to FIG. 4, an exemplary hypnogram 350 corresponding to the timeline 301 (FIG. 3), according to some implementations, is illustrated. As shown, the hypnogram 350 includes a sleep-wake signal 351, a wakefulness stage axis 360, a REM stage axis 370, a light sleep stage axis 380, and a deep sleep stage axis 390. The intersection between the sleep-wake signal 351 and one of the axes 360-390 is indicative of the sleep stage at any given time during the sleep session.

The sleep-wake signal 351 can be generated based at least in part on physiological data associated with the user (e.g., generated by one or more of the sensors 130 described herein). The sleep-wake signal can be indicative of one or more sleep stages, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 350 is shown in FIG. 4 as including the light sleep stage axis 380 and the deep sleep stage axis 390, in some implementations, the hypnogram 350 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration amplitude ratio, an inspiration-expiration duration ratio, a number of events per hour, a pattern of events, or any combination thereof. Information describing the sleep-wake signal can be stored in the memory device 114.

The hypnogram 350 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (t_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based at least in part on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based at least in part on, for example, data generated by the motion sensor 138, the microphone 140, the camera 150, or any combination thereof. The go-to-sleep time can be determined based at least in part on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

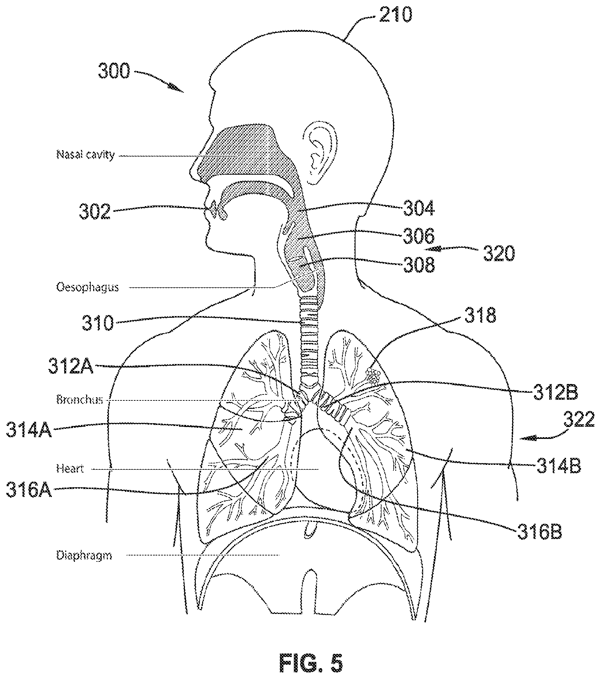

FIG. 5 shows an overview of an airway 300 of the user 210. The airway 300 includes, more specifically, a mouth 302, a throat 304 (also referred to as the oropharynx), a laryngopharynx 306, a larynx 308, a trachea 310, main bronchi 312A and 312B, lungs 314A and 314B, secondary bronchi 316A and 316B, and alveoli 318. The airway 300 forms a series of branching tubes, which become narrower, shorter, and more numerous as they penetrate deeper into the lungs 314A, 314B of the user 210. The prime function of the lungs is gas exchange, allowing oxygen to move from the inhaled air into the venous blood and carbon dioxide to move in the opposite direction. The trachea 310 divides into main bronchus 312A and main bronchus 312B, which in turn divide into the secondary bronchi 316A and the secondary bronchi 316B, located in the lungs 314A, 314B. The secondary bronchi 316A and 316B can branch out into higher order bronchi as well. The bronchi make up conducting airways, and do not take part in gas exchange. Further divisions of the airway 300 leads to the respiratory bronchioles, and eventually to the alveoli 318. The alveolated region of the lungs is where the gas exchange takes place, and is referred to as the respiratory zone. The laryngopharynx 306, the larynx 308 and the vocal folds are generally located in a neck area 320 of the user 210. The trachea 310, the main bronchi 312A and 312B, the lungs 314A and 314B, the secondary bronchi 316A and 316B, and the alveoli 318 are generally located in a thoracic cavity 322 of the user 210.

In order to measure the airway 300 of the user 210, various components of system 100 can be used to deliver one or more pressure oscillations to the airway 300. The acoustic signal will reflect off of various portions of the airway 300 of the user 210 and/or off of any obstructions in the airway 300. This reflection can be measured and used to determine the value of various parameters associated with the user 210 airway 300. These parameters can be indicative of the presence, identify, severity, etc. of various different health conditions. As discussed further therein, the acoustic signal can be generated by a transducer (e.g., a motor such as the blower motor of the respiratory therapy system 122, speaker, etc.) located anywhere in the system 100, including in the respiratory therapy device 122, the user interface 124, the conduit 126, or elsewhere. Reflections of the acoustic signal can be detected by a transducer (such as a microphone), which can in turn generate acoustic data representative of the reflections.

The term "acoustic signal" is used herein to refer to these pressure oscillations. In some implementations, the acoustic signal is an alternating signal with a varying amplitude, and can have any number of frequencies. In some implementations, the frequency of the acoustic signal is low enough that the acoustic signal is not audible by the user 210 or other individuals. However, the term "acoustic signal" is used herein to refer to any of the pressure oscillations delivered to the airway 300 of the user 210, whether the acoustic signal is audible to the user 210 or not.

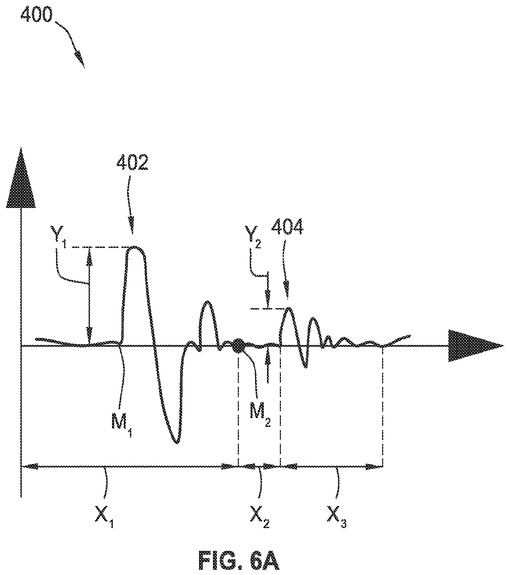

FIG. 6A is a cepstrum that illustrates an acoustic signal 400 with a varying acoustic amplitude along a Y-axis as the acoustic signal travel along an X-axis. The acoustic signal 400 has an acoustic amplitude that varies based generally on any encountered physical obstructions. The cepstrum of the acoustic signal 400 is obtained via one or more signal processing methods.

The traveled path along the X-axis includes a mask portion X1 that refers to a distance traveled by the acoustic signal 400 within a mask worn by the user. The mask is located at location M1. In describing the acoustic signal, the term "mask" is used to refer to the user interface that the user 210 is currently using. However, those of skill in the art understand that the description generally also applies to other types of user interfaces as well. In implementations where the transducer is not located in the mask (e.g., the transducer is located in the respiratory therapy device 122 or the conduit 126), the acoustic signal 400 will include a pre-airway portion that shows the travel of the acoustic signal 400 prior to reaching the mask at mask location M1.

At the mask location M1, the acoustic signal 400 has a first amplitude Y1 that is indicative at least in part of a first acoustic reflection 402, which is caused by the mask at the mask location M1. The mask location M1 ends at a mouth location M2. The distance between the mask location M1 and the mouth location M2 is indicated as X1. After encountering the mouth location M2, the acoustic signal 400 further travels into the airway 300 through a mouth portion X2, which represents the distance the acoustic signal 400 travels within the mouth 302 of the user 210. There, the acoustic signal 400 encounters a physical obstruction P. At the physical obstruction P, the acoustic signal has a second amplitude Y2 that is indicative at least in part of a second acoustic reflection 404 (also referred to as "the acoustic reflection 404"), which is caused by the physical obstruction P located within the throat 304 of the user 210. The physical obstruction P extends into the airway 300 a third distance X3 after the second distance X2. Thus, the cepstrum identifies a distance (e.g., the first distance X1, the second distance X2, and or the third distance X3) that is associated with the first and second acoustic reflections 402, 404, the distance showing the location of the physical obstruction P within the airway 300 of the user 210.

The first acoustic reflection 402 has a specific signal morphology that is different than the signal morphology of the second acoustic reflection 404. For example, the first amplitude Y1 is greater than the second amplitude Y2. In another example, the shape of the first acoustic reflection 402 is different than the shape of the second acoustic reflection 404. According to some examples, the signal morphology includes an acoustic shape of an acoustic wave. According to some implementations of the present disclosure, one or more of the physical characteristics of the physical obstruction P can be estimated based on the signal morphology of one or more of the first acoustic reflection 402 and the second acoustic reflection 404.

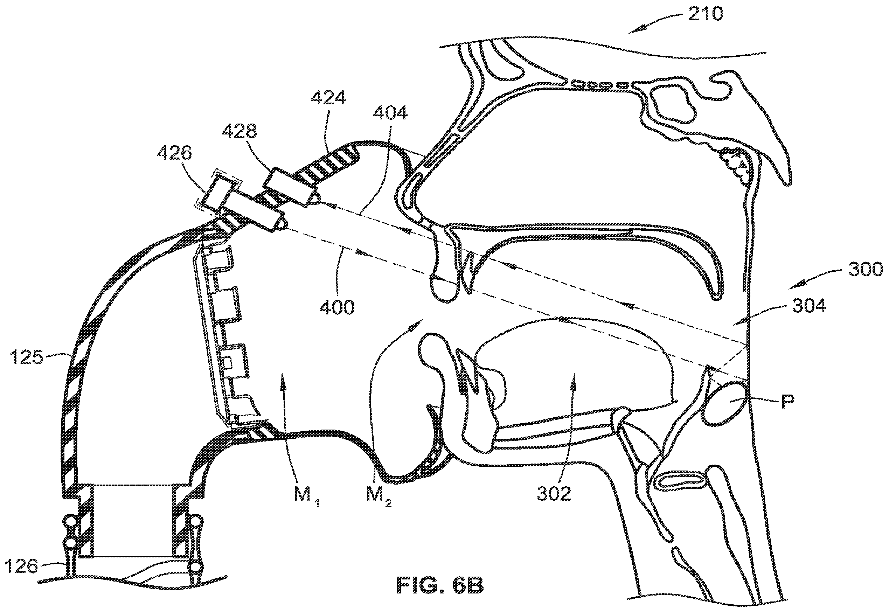

FIG. 6B illustrates an implementation where the user interface worn by the user 210 is a full facial mask 424, and where the transducer that generates the acoustic signal 400 is located in the mask 424. The mask 424 covers both the mouth and the nose of the user 210. The mask 424 is connected to the conduit 126 via an elbow connector 125. The other end of the conduit 126 can be connected to the respiratory therapy device 122. Together, the conduit 126, the elbow connector 125, and the mask 524 form the air pathway that fluidly couples the respiratory therapy device 122 and the airway 300 of the user 210.

The mask 524 includes a speaker 426 and a microphone 428. The speaker 426 is the transducer that generates the acoustic signal, and the microphone 428 is the transducer that generates acoustic data representative of the reflections of the acoustic signal. The speaker 426 can be the speaker 142 of system 100, or can be an additional or alternative speaker. Similarly, the microphone 428 can be the microphone 140 of system 100, or can be an additional or alternative microphone. The speaker 426 is configured to generate the acoustic signal 400 that is delivered into the airway 300 of the user 210. The microphone 428 is configured to generate acoustic data that is representative of any reflections of the acoustic signal 400 caused by (i) a portion of the airway of the individual, (ii) an obstruction within the airway of the individual, or (iii) both (i) and (ii).

In the illustrated implementations, the speaker 426 and the microphone 428 are disposed in separate housings. In other implementations, the speaker 426 and the microphone 428 can be disposed in a single housing, forming an acoustic device or sensor. In some implementations, the speaker 426 and the microphone 428 are embedded in apertures defined in the mask 424. In other implementations, the speaker 426 and the microphone 428 are integrally formed with the mask 424.

In some implementations, the speaker 426 generates the acoustic signal 400 in the form of a sound, the sound being one or more of a standard sound (e.g., an original, unmodified sound from an off-the-shelf sound application), a custom sound, an inaudible frequency, an audible frequency, a white noise sound, a broad band impulse, a continuous tone, a pulsed tone, a sinusoidal waveform, a square waveform, a sawtooth waveform, a frequency modulated sinusoid (e.g., chirp), a continuous wave (CW) sound, and an ultra-wideband (UWB) sound (which could be white noise generated by a motor (such as the blower motor), an impulse generated by a speaker, a spread spectrum generated by a speaker, etc.). Other techniques could also be used to adjust the acoustic signal 400, such as adaptive frequency range gating, adaptive frequency range hopping, frequency shift keying, phase shift keying, etc. In some implementations, as discuss herein, the acoustic signal 400 can be calibrated, for example based on the type of user interface that the user is wearing, or other features related to the user and/or the system 100.

Generally, any type of frequency- or amplitude-modulated signal (such as a frequency-modulated continuous-wave) can be used, as well as unmodulated signals. The acoustic signal 400 can have a spread spectrum of frequencies, a pseudorandom spectrum of frequencies, or other forms. According to some other implementations, the acoustic signal 400 is in the form of one or more of an audible sound or an ultrasonic sound. Generally, the acoustic signal 400 can have any characteristics to ensure that the acoustic signal 400 is able propagate to a desired location within the user's airway and to ensure that the reflections of the acoustic signal 400 can be measured, based on any number of internal factors (e.g., physical features of the user) and external factors (e.g., the type of user interface that the user is wearing).

The speaker 426 and the microphone 428 can be coupled to a control system and a memory device (such as control system 110 and memory device 114). The control system is configured to operate both the speaker 426 and the microphone 428. The control system causes the speaker 426 to emit the acoustic signal 400, and causes the microphone 428 to generate the acoustic data representative of the reflections of the acoustic signal 400. The acoustic data can then be stored in the memory device.

As shown in FIG. 6B, the acoustic signal 400 (represented by a dashed line connecting a series of arrows) is directed towards the airway 300 of the user 210 by the speaker 426. Specifically, the acoustic signal 400 is directed through the mouth 302 of the user 210 and toward the back of the throat 304 of the user 210. When the acoustic signal 400 reaches the physical obstruction P in the back of the throat 304 of the user 210, the reflection 404 is directed back toward the mask 424, where the reflection 404 is detected by the microphone 428, and acoustic data representative of the reflection 404 is generated. The physical obstruction P can generally be any physical obstruction, such as the collapse of the airway 300 during an apnea event or a hypopnea event, an obstruction caused by some other respiratory condition (e.g., mucus buildup due to respiratory infection, etc.). While FIG. 6B shows the acoustic signal 400 being generally directed toward the mouth 302 of the user 210, in additional or alternative implementations, the acoustic signal 400 can be directed toward the nose of the user 210.

The acoustic data associated with the acoustic reflection 404 is analyzed and the physical obstruction P can be characterized. The characterization of the physical obstruction P is based at least in part on the analyzed acoustic data. According to some implementations of the present disclosure, the characterization is based solely on the analyzed acoustic data. The physical obstruction P can be indicative of an apnea event or a hypopnea event in the user 210. According to some implementations of the present disclosure, the acoustic data includes a physical measurement of at least one of a mouth area, a nose area, and a throat area of the user 210.

The characterization of the physical obstruction P includes, for example, determining a location of the physical obstruction P within the airway 300 of the user 210. According to the illustration of FIG. 6B, the physical obstruction P is after the second distance X2 from the point of entry of the acoustic signal into the user's airway 300, e.g., the mouth 302 of the user 210. The characterization further includes, according to other examples, determining the site size, site shape, and/or any other physical characteristics of the physical obstruction P.

According to some implementations of the present disclosure, one or more of the physical characteristics of the physical obstruction P are estimated based on a change in acoustic impedance, as indicated by the analyzed acoustic data. According to some implementations of the present disclosure, one or more of the physical characteristics of the physical obstruction P are estimated based on analyzing the acoustic reflection 404 that occurs from the physical obstruction P within the airway 300 of the user 210.

According to some implementations of the present disclosure, one or more of the physical characteristics of the physical obstruction P are estimated based on an analysis of (a) one or more acoustic waves reflecting at the mouth location M2 (before an entry into the airway 300 of the user 210), and (b) one or more acoustic waves reflecting at the physical obstruction P (after the entry into the airway 300 of the user 210). According to some other implementations of the present disclosure, one or more of the physical characteristics of the physical obstruction P are estimated based on the second distance X2 that acoustic signal travels into the airway 300 of the user 210 past the mouth location M2. According to some examples, the entry point into the airway 300 is the mouth 302 (e.g., at the mouth location M2) or a nose of the user 210.

According to some implementations of the present disclosure, one or more of the physical characteristics of the physical obstruction P are estimated based on one or more characteristics of the user 210, such as age, sex, weight, mouth size, neck size, or type of mask currently used by the user 210.

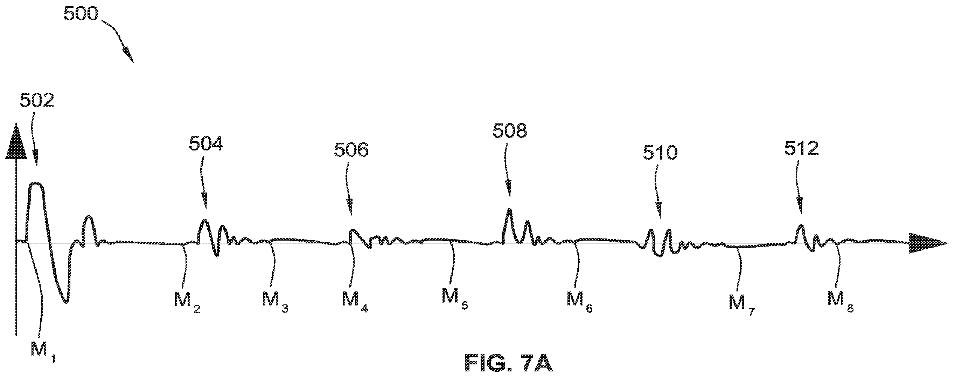

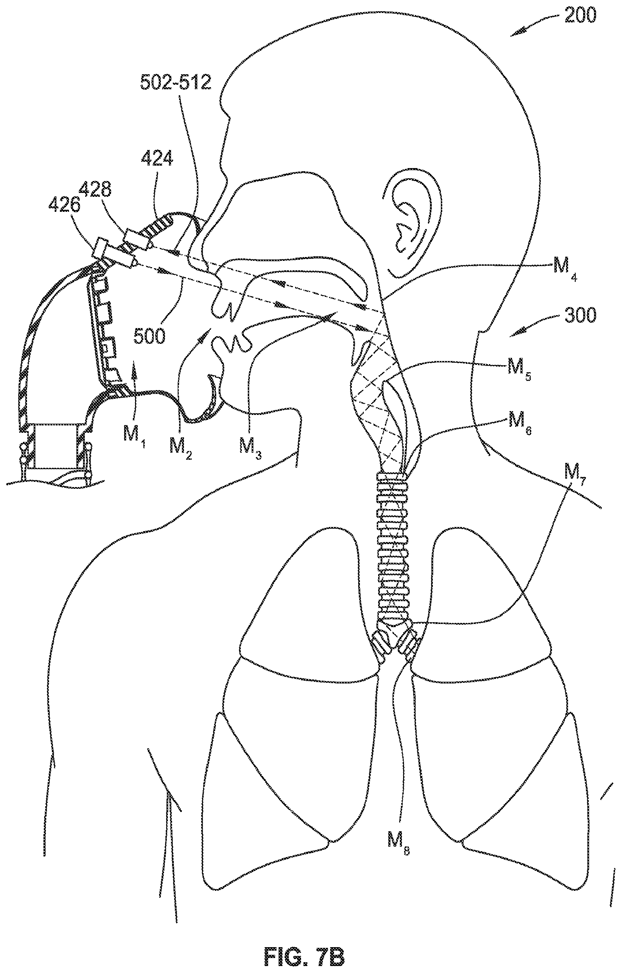

Referring now to FIGS. 7A and 7B, the acoustic signal emitted by the speaker 426 can also be used to characterize and/or measure portions of the airway 300 beyond the throat 304 of the user 210, such as the laryngopharynx 306, the larynx 308, the trachea 310, various bronchi, and the lungs 314A, 314B. FIG. 7A is a cepstrum that illustrates an acoustic signal 500 directed toward the airway 300 of user 210. Acoustic signal 500 is generally the same as or similar to acoustic signal 400, and has a varying amplitude along the Y-axis as the acoustic signal travels along the X-axis. The cepstrum includes the mask portion starting at mask location M1, one or more reflections 502 (similar to reflection 402) within the mask, and a mouth portion starting at mouth location M2. However, as shown in FIG. 7B, there is no physical obstruction P which the acoustic signal 500 reflects off of, and the acoustic signal 500 continues traveling through the airway 300. The acoustic signal 500 travels from the back of the mouth 302 at location M3 to the back of the throat 304 at location M4, which can result in one or more reflections 504.

The acoustic signal 500 can then continue to propagating through the airway 300. The acoustic signal 500 travels through the laryngopharynx 306, from location M4 at the back of the throat 304 to the beginning of the larynx 308 at location M5. A portion of the acoustic signal 500 can reflect off of a portion of the laryngopharynx 306, resulting in one or more reflections 506.

Next, the acoustic signal 500 travels through the larynx 308, from location M5 to location M6 at the beginning of the trachea 310. A portion of the acoustic signal 500 can reflect off of a portion of the larynx 308, resulting in one or more reflections 508. The acoustic signal 500 next travels through the trachea 310, from location M6 at the top of the trachea 310 to location M7 at the bottom of the trachea 310, where the trachea 310 splits into the main bronchi 312A and 312B. A portion of the acoustic signal 500 can reflect off of a portion of the trachea 310, resulting in one or more reflections 510. Finally, the acoustic signal 500 can travel through the main bronchi 312A, 312B, from location M7 to location M8 at the lungs 314A, 314B. A portion of the acoustic signal 500 can reflect off of a portion of the main bronchi 312A, 312B, resulting in one or more reflections 512.

The reflections 502-512 can propagate back through the airway 300 of the user 210 to the microphone 428. Acoustic data that is representative of the reflections 502-512 can be generated by the microphone 428 and stored in the memory device (such as memory device 114 of system 100). The acoustic data can then be analyzed to determine various parameters of the airway 300, such as acoustic impedance, resonant frequency, location and size of any physical obstructions, and others.

While FIG. 7A shows a cepstrum of an acoustic signal with specific reflections, and FIG. 7B shows a specific path the acoustic signal can take through the airway 300 of the user 210, those of skill in the art will understand that the acoustic signal can travel along a variety of different paths once it is directed into the airway 300. The airways of different users can have different physical characteristics, which can result in a wide variety of acoustic data. For example, in one user, the acoustic signal may generate reflections at multiple points within the laryngopharynx, while in another user the acoustic signal may only generate a reflection at single point within the laryngopharynx. However, for both users, acoustic data indicative of those reflections and of various characteristics of the user's airway can be generated.

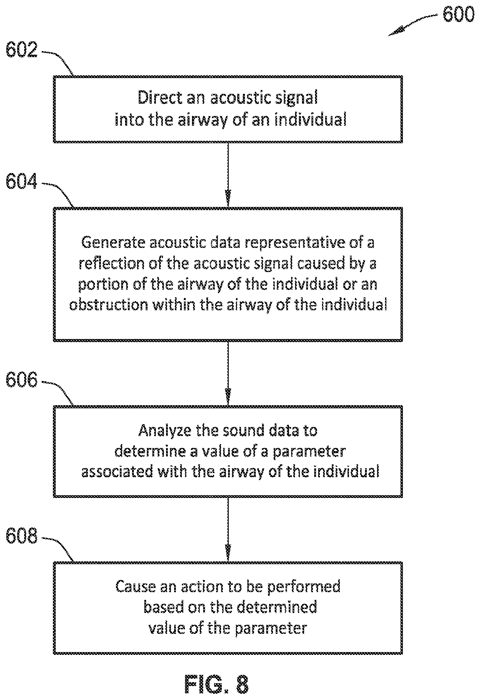

FIG. 8 illustrates a method 600 for monitoring a user. Method 600 can be used, for example, to identify a respiratory condition of the user, prior to the respiration condition manifesting externally-identifiable physical symptoms. Method 600 can be implemented, for example, using a system (such as system 100) that includes a respiratory therapy system (such as respiratory therapy system 120) having a respiratory therapy device configured to supply pressurized air (such as respiratory therapy device 122), a user interface (such as user interface 124) coupled to the respiratory therapy device via a conduit (such as conduit 126). The user interface is configured to engage with the user, and aids in directing the pressurized air to the user's airway. Implementation of method 600 can also use a memory (such as memory device 114) that stores machine-readable instructions, and a control system including one or more processors (such as control system 110 and processor 112) that executed the machine-readable instructions to execute the steps of method 600.

Step 602 of method 600 includes directing an acoustic signal into the airway of the user. The acoustic signal can be generated by a transducer, such as speaker 426 located in the user interface (e.g., within the interior of a face mask). The acoustic signal may be directed toward the airway of the user by aligning the output of the transducer with the user's airway. In other implementations, the user interface could include various physical structures that aid in directing the acoustic signal toward the user's airway, once emitted by the speaker 426.

The acoustic signal can have a variety of different shapes, including a varying amplitude repeated at a given frequency. The frequency of the acoustic signal can be between about 0 and about 100 Hertz (Hz), between about 0 and about 50 Hz, between about 0 and about 25 Hz, or other ranges. In some implementations, the frequency is greater than about 0 Hz and less than about 4 Hz, or other ranges. Generally, lower-frequency acoustic signals (between about 0 and 5 Hz) can propagate out to the periphery of the lungs (e.g., the higher-order bronchi), while higher-frequency acoustic signals (around 20 Hz) can only propagate to the main bronchi.

One or both of the amplitude and the frequency of the acoustic signal can be modulated as needed. In some implementations, the frequency of the acoustic signal that is directed into the airway of the user is increased and/or decreased in a step-wise fashion. For example, the acoustic signal could initially be emitted with a frequency of about 0.1 Hz, and be increased in increments of about 0.1 Hz to about 2 Hz over a time period of about ten seconds. Alternatively, the frequency of the acoustic signal can be increased in a continuous fashion. More generally, the acoustic signal can have any desired characteristics. In some implementations, the characteristics of the acoustic signal (such as the frequencies, amplitudes, waveforms, etc.) can be manually selected by an individual. In other implementations, the characteristics of the acoustic signal may be automatically selected via machine-executable instructions executed by a control system (such as the control system 110.

In some implementations, the characteristics of the acoustic signal are based on the type of user interface that is worn by the user. As discussed herein, a variety of different types of user interfaces may be worn by the user, including a facial mask, a nasal mask, a nasal pillow mask, etc. Different types of user interfaces can affect the acoustic signal in different ways. For example, a facial mask may attenuate the acoustic signal more than a nasal mask or a nasal pillow mask. Thus, when using a given acoustic signal, the reflections of the acoustic signal may result in acoustic data with a signal-to-noise ratio that is too low to yield an accurate measurement of the parameter being measured, depending on the type of user interface being worn by the user. Step 606 can therefore include determining the type of user interface being worn by a user prior to directing the acoustic signal into the user's airway.

In some implementations, the system that implements the method 600 (such as system 100) can receive input from the user indicating what type of user interface the user is wearing. In other implementations, the system that implements the method 600 can determine what type of user interface that the user is wearing without receiving input from the user. For example, the system may include an image sensor (such as camera 150) that generates image data of the user wearing the user interface. The control system can analyze the image data to determine what type of user interface that the user is wearing. In another example, the system may direct a preliminary acoustic signal into the user's airway. Based on the reflections of the acoustic signal, the system can determine the type of user interface that the user is wearing, and then determine the characteristics of the acoustic signal that will be used in step 602. In still further implementations, the frequency of the acoustic signal is swept over a wide range of frequencies, amplitudes, waveforms, or any combination thereof, to ensure that at least some portion of the reflections of the acoustic signal result in acoustic data that has a sufficiently large signal-to-noise ratio so that the respiratory parameter can be accurately measured.

As noted, in some implementations, the transducer emitting the acoustic signal can be the speaker 426 that is located in the user interface. In these implementations, the user interface will generally be a full facial mask that covers the user's mouth and nose, but other user interfaces could also be used. In other implementations, the transducer can be located at some other part of the system (which could be system 100) can be used. The transducer could be located in the respiratory therapy device, in the conduit, or in any other suitable location. In implementations where the transducer is located within the respiratory therapy device, the transducer can be located within a housing of the respiratory therapy device alongside the motor used to generate the pressurized air. In some implementations, as noted, the transducer is a speaker. However, in other implementations, the transducer can be a motor, such as the motor of the respiratory therapy device that generates the pressurized. In this implementation, the noise of the motor as it operates is used as the acoustic signal, which is directed to the user's airway through the conduit of the respiratory therapy system. In still other implementations, the conduit, the elbow connector, or the user interface can include a separate device that can be driven by the pressurized air from the motor of the respiratory therapy device. When driven by the pressurized air, the device generates the required acoustic signal and directs to the user's airway.

Step 604 of method 600 includes generating acoustic data that is representative of one or more reflections of the acoustic signal. As described herein, as the acoustic signal propagates through the user's airway, the acoustic signal can reflect off portions of the user's airway and/or any obstructions within the user's airway. The reflection can be detected by a transducer, which generates the acoustic data representative of the reflections. In some implementations, the transducer is a microphone, and can be located in the user interface (such as microphone 428 located in the mask). The microphone could be located at other locations within the system, such as within the conduit, within the respiratory therapy device, within a connector (such as an elbow connector) between the user interface and the conduit, etc. The acoustic data generated by the transducer is generally representative of any characteristics of the reflections that are needed to analyze the user's airway. For example, the acoustic data can be representative of the amplitude of the reflections, the physical distance between the reflections, the temporal length between the reflections, the frequency of a series of reflections, etc.

At step 606 of method 600, the generated acoustic data is analyzed to determine a value of a parameter associated with the user's airway. The analysis of the data can be performed by the control system. The parameter can be any parameter that can be used to characterize the user's airway. For example, the parameter could be the impedance of the user's airway (which is a combination of the resistance of the airway and the reactance of the airway, both of which could also be measured parameters), a resonant frequency of the user's airway, a location of any physical obstructions in the user's airway, a size of any physical obstructions in the user's airway, or any other parameter of interest. In some implementations, various signal processing techniques can be applied to obtain data of interest. Time or frequency spectrograms can be obtained from the acoustic data, which can be used to analyze the user.

Generally, the value of the parameter is indicative of whether the user is suffering from any type of respiratory-associated disease or condition. For example, the respiratory impedance is a combination of both the energy required for the acoustic signal to propagate through the user's airway (the respiratory resistance) and the amount of recoil by the tissue generated against the acoustic signal (the respiratory reactance). These characteristics of the user's airway can change in the presence of a respiratory condition. By measuring the respiratory impedance, the changes in the user's airway due to the presence of the respiratory condition can be detected, often earlier than external physical symptoms may manifest. Thus, the respiratory condition can be detected early, which provides more options for treatment. Possible respiratory conditions that can be detected include obstructions, pneumonia, inflammation, allergies, lung cancer, bacterial infection, viral infection, a cold, asthma, chronic obstructive pulmonary disease (COPD), cystic fibrosis, congestive heart failure, pneumothorax (collapsed lung), SARS-CoV-2, and others. With some of these respiratory conditions, there can be increased pulmonary resistance in the bronchioles, which reduces the amount of air inhaled in each breath, and subsequently the amount of oxygen that reaches the pulmonary arteries. This deficiency can thus be measured using method 600, which can provide an early indication to the user of the respiratory condition.

In some implementations, the acoustic data can be windowed to remove unneeded acoustic data. For example, depending on where the transducer is located and thus where the acoustic signal originates, some of the reflections of the acoustic signal may not associated with reflections off any portion of the user's airway. Instead, these reflections could be the result of the acoustic signal interacting with the conduit and/or the user interface, and thus cannot be used to measure the parameter associated with the user's airway. Thus, these portions of the acoustic data can be discarded prior to determining the value of the parameter.

As noted herein, the type of user interface that the user wears can affect the acoustic data. If the type of user interface being used is known, the acoustic data can be adjusted to account for these variations, and to obtain an accurate value for the parameter. Sensors (such as sensors 130) can be used to aid in determining the type of user interface the user is wearing. These sensors could include an acoustic sensor and/or a microphone (such as acoustic sensor 141 and microphone 140), a camera (such as camera 150), and other sensors.

At step 608 of method 600, an action is performed based on the determined value of the parameter. In some implementations, the action can include comparing the value of the parameter to a baseline value of the parameter, to aid in determining whether the user has a respiratory condition, or whether the user's respiratory condition has become worse. Further action can then be taken. In some examples, the baseline value is an expected value based on various characteristics of the user, including age, sex, gender, ethnicity, location, known medical conditions, height, weight neck circumference, and others. The expected value could be a previously-determined value of the parameter from an individual sharing one or more characteristics of the user. The expected value could also be based on one or more averages of multiple different individuals sharing different characteristics with the user. In some implementations, the value of the parameter deviating from the baseline value by a certain amount indicates that the user is suffering from a specific respiratory condition. In other implementations, the value of the parameter deviating from the baseline value by a certain amount indicates that the user is suffering from an unknown respiratory condition, and that further follow-up is needed to determine the identity of the respiratory condition.

The baseline value could also be previously determined from an individual that was diagnosed with a specific respiratory condition. By comparing the determined parameter value of the user with the previously-determined parameter value from the individual diagnosed with the specific respiratory condition, and can be determined whether the user is currently suffering from that same specific respiratory condition. Further, by comparing the parameter value of the user with previously-determined parameter values from multiple different individuals known to have different respiratory conditions, the identity of the user's respiratory condition can be determined.

In other implementations, the baseline value of the parameter is a value of the parameter that was previously determined for that same user. By monitoring the value of the parameter for the user over a time period, any progression of the respiratory condition can be tracked, and appropriate intervention can be triggered if warranted. The value of the parameter can be monitored over a day, a week, a month, a year, or any other suitable time period. Thus, the baseline value of the parameter of the user can be determined at a first time, and the current value of the parameter can be later determined at a second time after the first time. This analysis technique allows the parameter to be monitored over time to determine long-term trends. If the trends indicate that a certain condition is worsening, this can be flagged for the user or an appropriate third party, such as a spouse or a healthcare provider.

In some implementations, the time difference between the current parameter value and the baseline parameter value is selected based on what parameter is being measured and/or what respiratory condition is being monitored. For example, to monitor a user's allergies, the value of the parameter determined during springtime (e.g., a first season of the year) could be compared to the value of the parameter measured during the previous fall or winter (e.g., a second season of the year), to see if the user is suffering from any allergies, or is likely to suffer from allergies during the spring. The value of the parameter could also be measured across successive spring seasons, to see if the user's allergies are getting worse over time.

The action can also include combining the determined value of the parameter with other sources of data to aid in determining whether the user has a respiratory condition, or is at risk of developing a respiratory condition. For example, if the user's medical record indicates that they have previously been diagnosed with a respiratory condition, a given value of the parameter may more strongly suggest the current presence of that respiratory condition or another respiratory condition, as compared a user who has not been previously diagnosed with the respiratory condition. Other data can also be utilized to aid in determining whether the determined value of the parameter is indicative of the present of a respiratory condition, such as age, sex, gender, ethnicity, location, known medical conditions, height, weight, neck circumference, and others. This data could be obtained from the user's medical records, but additionally or alternatively could be obtained directly from the user. For example, the user could fill out a questionnaire or otherwise provide certain data that can aid in determining the presence and identify of a potential respiratory condition.

Additionally, data from other sources can also be used. For example, data from any sensors (such as sensors 130) can be used to aid in evaluating the acoustic data. For example, the data from the sensors could be used to measure the user's breathing rate, and determining if the user is breathing abnormally. This determination can be combined with the value of the parameter to provide more insight into whether the user is suffering from a respiratory condition. Additional data could also be obtained from smart devices, such as a smart watch or other wearable, a smart speaker, a smart inhaler, etc. This data could include pulse oximetry data and/or heart rate data obtained from the smart watch or other wearable. In still other implementations, the additional data includes data associated with the user of medication. For example, users with asthma may use a smart inhaler, which can track the user's use of the inhaler and different medicines (e.g., corticosteroids, bronchodilators). Data representing these uses can be used to aid in determining the presence, identify, severity, etc. of any potential respiratory conditions. In still further implementations, the additional data includes data generated by medical measurement devices, such as a pulse oximeter, a blood pressure measurement device (e.g., a blood pressure cuff), a thermometer, a heart rate monitor, an analyte measurement device (e.g., a blood glucose meter), or others.

In some implementations, the action can include activating certain functions of the respiratory therapy system. For example, if the value of the parameter indicates that the user has a respiratory condition, a medicament designed to be inhaled can be injected into the air pathway along with the pressurized air, so as to deliver the medicament to the user's airway. The medicament could be a bronchodilator, or another medicament designed to aid the user in recovering from the respiratory condition.

In some implementations, the action can include adjusting various settings of the respiratory therapy system. The respiratory therapy system may be being used normally as a positive airway pressure system. Based on the value of the parameter that is measured, the pressure and/or the flow rate of the pressurized air supplied by the respiratory therapy system can be modified. For example, if the measured value of the parameter indicates that the respiratory impedance of the user is higher than expected, the pressure of the pressurized air can be increased. In another example, humidification (e.g. steam) can be added to the pressurized air supplied to the user's airway.

In some implementations, the respiratory therapy system could also be adjusted so as to operate in a different manner. For example, the respiratory therapy system could initially be operated as a CPAP system, but then may be modified to operate as a BPAP system. The respiratory therapy system could also be modified to operate as an oxygen concentrator or a ventilator.

These implementations can also be used to monitor the effectiveness of the respiratory therapy system itself. For example, the value of the parameter can be determined when the respiratory therapy system is initially begun to be used as a CPAP system with certain pressure and flow settings. After a predetermined time period (such as an hour, a week, etc.), the value of the parameter can again be determined to see if the use of the CPAP system has improved the user's respiratory condition. If it has not, the settings of the respiratory therapy system can be modified. Thus, in some implementations, the system is a closed-loop system that can analyze data related to its own performance, and adjust its settings accordingly.

In related implementations, the respiratory therapy system can also be used to monitor the user's recovery from a respiratory condition, in addition to or alternatively to monitoring the development of a respiratory condition. In these implementations, the value of the parameter can be determined periodically during or following treatment of the respiratory condition (for example during a medication regimen, after an inhaler use, etc.). The effectiveness of the treatment can thus be monitored using the techniques disclosed herein, which can provide an early indication of whether the treatment is working, or whether a different treatment may need to be tried.

In some implementations, the action can include generating a notification and/or a report, and transmitting the notification and/or report to the user or to a third party. If the value of the parameter indicates that the user has a respiratory condition or appears to be developing a respiratory condition, the user can be notified of this fact, for example via a text message, a phone call, an email, or via text displayed on an electronic display device able to communicate with the respiratory therapy system and other associated components, such as sensors. In some implementations, the notification is just an indication that the user has developed or is developing the respiratory condition. In other implementations, the user is sent a full report that includes a variety of information related to the respiratory condition. The third party that the notification and/or report is sent to can include the user's spouse or significant other, a family member, a friend of the user, a caretaker, a healthcare provider, or others. In some implementations, any data associated with method 600 (including acoustic data and other data used) can be stored as part of the user's medical record. In other implementations, a recommendation for therapy can be transmitted to the user or to a third party. The recommendation for therapy can include a recommendation to adjust the settings of the respiratory therapy device to aid in treating the condition, and recommendation to take medicine, a recommendation to schedule an appointment with a healthcare provider (for example to undergo a more thorough examination of the user's airway), and other recommendations.

In some implementations, instructions can be provided to the user prior to the initiation of method 600, for example via a user interface (such as user interface 124) of the system. For example, for certain users, determining the value of the parameter may be easier or more accurate when the user is in a certain physical position or orientation. The system can instruct the user to move to the specified physical position or orientation, which could be a standing position, a seated position, an inclined position, a laying position, or others. One or more sensors (such as the motion sensor 138 and/or the camera 150) can determine when the user has moved to the specified physical position or orientation. The system can subsequently begin to direct the acoustic signal into the user's airway. In another example, the instructions may direct the user to breath according to a specified pattern during some or all of the execution of method 600. The instructions can direct the user to hold their breath, breath slowly, breath rapidly, breathe deeply, breathe shallowly, or other patterns. In some implementations, the user could also be instructed to forcefully inhale or exhale. The resulting flow of air can be measured (for example using the microphone 428 and/or any of the sensors 130) to provide additional data related to a respiratory condition of the user.

As noted herein, method 600 can be implemented using a respiratory therapy system that the user may already be using at night while they sleep, such as a CPAP system. Because the user is already wearing the user interface at night, providing the speaker 426 and the microphone 428 in the user interface is a simple and efficient update that allows the parameter to be measured on a consistent schedule, allowing for potentially earlier detection of respiratory conditions. In some implementations, method 600 can be executed at the beginning of the sleep session (e.g., at night when the user gets into bed), when the user initially dons the user interface. When this occurs, the system can instruct the user to breath normally, and the acoustic signal can be directed into the user's airway so that the value of the parameter for that sleep session can be determined.

In additional or alternative implementations, method 600 can be executed at the end of every sleep session (e.g., in the morning when the user wakes up) to determine the value of the parameter. In still other implementations, method 600 can be executed both at the beginning and the end of one or more sleep sessions. In yet other implementations, method 600 can be executed in the middle of the sleep session to measure the value of the parameter. The execution of method 600 in the middle of the sleep session could be random, or could be based on data related to the user's use of the respiratory therapy system during the sleep session.

In still other implementations, method 600 can be executed to briefly interrupt the standard operation of the respiratory therapy system. For example, the respiratory therapy system can be normally operating as a CPAP system (for example during the sleep session). The operation of the motor to provide positive pressure to the user's airway can briefly be stopped so that the acoustic signal can be directed to the user's airway and the parameter can be measured. Once the reflections of the acoustic signal have been received, the motor can resume normal operation. This timing can include implementations where a separate speaker is used to generate the acoustic signal, and in implementations where the motor itself is used to generate the acoustic signal.

Method 600 can also include separately directing the acoustic signal into the user's airway via both the user's mouth and the user's nose. The acoustic data can be analyzed to separate the two measurements. In some of these implementations, airflow through either the mouth or nose can temporarily be blocked off (for example via a smart mask) so as to measure the other of the mouth or nose.

The temporal duration of method 600 can also be limited so as to not excessively disrupt or annoy the user. For example, in some implementations, operation of the transducer to produce the acoustic signal (whether the transducer is a speaker in the user interface, the motor of the respiratory therapy device, or another transducer) can be relatively loud, so the transducer may be operated only for a short enough period to generate a needed amount of acoustic data.

In some implementations, the various steps of method 600 can also be implemented using respiratory therapy systems other than typical respiratory therapy systems used by users with SDB, such as CPAP systems or BIPAP systems. For example, some users may use certain daytime breathing assistance systems, such as supplemental oxygen, high flow nasal oxygen (HFO), heated humidified high-flow oxygen (HHHF), and others. These systems often include some type of user interface that the user wears, and a conduit to delivery air to the user interface and the user. These systems can be used to direct an acoustic signal into the user's airway and measure the resulting reflections, in order to determine the value of various parameters.

In some implementations, the method 600 includes determining whether an obstruction is an upper airway obstruction or a lower airway obstruction. A lower airway obstruction can be indicative of infection and/or inflammation, for example due to pneumonia, asthma, etc. Lower airway obstructions can often be difficult to detect and treat using conventional devices. By directing an acoustic signal into the user's airway that can propagate into the user's lower airway, potential obstructions in the lower airway can be detected. And because these techniques can be used with a wide variety of respiratory therapy systems, users can both treat upper and lower airway obstructions, and detect and treat other respiratory conditions.

In some implementations, method 600 can be used as a diagnosis/screening tool. If method 600 indicates the presence (or the potential future presence) of a respiratory condition, the user can decide to visit with their healthcare provider for follow-up care. In these implementations, method 600 could be performed on the user at any desired time, regardless of how method 600 is implemented. For example, even if method 600 is implemented using a respiratory therapy system that normally operates at night during a sleep session, the user could utilize the respiratory therapy system during the day to implement method 600. In other implementations, method 600 can be used as part of a treatment routine. For example, as discussed herein, in response to the measured parameter indicating the presence (or the potential future presence) of a respiratory condition, the administration of a medicament (or other therapy used to treat the respiratory condition, such a humidification, a bronchodilator, etc.) can automatically be triggered.

Generally, method 600 can be implemented using a system having a control system with one or more processors, and a memory storing machine readable instructions. The controls system can be coupled to the memory, and method 600 can be implemented when the machine readable instructions are executed by at least one of the processors of the control system. Method 600 can also be implemented using a computer program product (such as a non-transitory computer readable medium) comprising instructions that when executed by a computer, cause the computer to carry out the steps of method 600.

While the present disclosure has been described with reference to one or more particular embodiments or implementations, those skilled in the art will recognize that many changes may be made thereto without departing from the scope of the present disclosure. Each of these implementations and obvious variations thereof is contemplated as falling within the scope of the present disclosure. It is also contemplated that additional implementations according to aspects of the present disclosure may combine any number of features from any of the implementations described herein.

The present invention is defined by the claims which follow.

## Claims

1. A system (100) for monitoring an airway of an individual, the system comprising:
a respiratory therapy system (120) including:
a respiratory therapy device (122) configured to supply pressurized air; and
a user interface (124) coupled to the respiratory therapy device (122) via a conduit (126), the user interface (124) being configured to engage the individual and aid in directing the supplied pressurized air to the airway of the individual;
a memory (114) storing machine-readable instructions; and
a control system (110) including one or more processors (112) configured to execute the machine-readable instructions to:
cause an acoustic signal to be directed, via the conduit (126) and the user interface (124), into the airway of the individual;
generate acoustic data representative of one or more reflections of the acoustic signal caused by (i) a portion of the airway of the individual, (ii) an obstruction within the airway of the individual, or (iii) both (i) and (ii);
analyze the acoustic data to determine a value of a parameter associated with the airway of the individual;
wherein the one or more processors (112) of the control system (110) are further configured to execute the machine-readable instructions to:
receive additional data associated with the individual;
modify the value of the parameter based on the additional data; and
based on the modified value of the parameter and the additional data, cause an action to be performed.

2. The system (100) of claim 1, wherein the parameter is an impedance of the airway of the individual.

3. The system (100) of claim 1, wherein the parameter is an impedance of the airway of the individual, a resonant frequency of the airway of the individual, a location of the physical obstruction, a size of the physical obstruction, or any combination thereof.

4. The system (100) of any one of claims 1 to 3, wherein the action includes causing a medicament to be injected into the air pathway, causing a notification or report to be transmitted or displayed to the individual or to a third party, generating a recommendation for therapy, adjusting a pressure, a flow rate, or both, of the pressurized air supplied to the airway of the individual, or any combination therein; and/or
the pressurized air is supplied to the individual of the airway at a first pressure, and the action includes causing the respiratory therapy device (122) to supply the pressurized air to the airway of the individual at a second pressure that is greater than the first pressure.

5. The system (100) of any one of claims 1 to 4, wherein the respiratory therapy system (120) initially operates as a positive airway pressure system, and wherein the action includes adjusting the respiratory therapy system (120) such that the respiratory therapy system (120) operates as an oxygen concentrator, a ventilator, or both.

6. The system (100) of any one of claims 1 to 5, wherein the one or more processors (112) of the control system (110) are further configured to execute the machine-readable instructions to compare the determined value of the parameter with a previously-obtained baseline value of the parameter, and optionally wherein:
the action is based on the comparison between the determined value of the parameter and the previously-determined baseline value of the parameter, and/or
the baseline value of the parameter is previously obtained from the individual at a first time, and wherein the control system is configured to execute the machine-readable instructions to determine the value of the parameter at a second time that is after the first time, optionally wherein a time difference between the first time and the second time is a day, a week, a month, or a year, or wherein the first time is during a first season of the year, and wherein the second time is during a second season of the year; and/or
the baseline value of the parameter is previously obtained from another individual sharing one or more characteristics with the individual, optionally wherein the one or more characteristics include age, sex, gender, ethnicity, location, medical conditions, height, weight, neck circumference, or any combination thereof; and/or
the baseline value of the parameter is previously obtained from another individual diagnosed with a respiratory-associated disease or condition, and wherein the comparison between the determined value of the parameter and the baseline value of the parameter is indicative of whether the individual has the respiratory-associated disease or condition.

7. The system (100) of any one of claims 1 to 6, wherein the value of the parameter is indicative of whether the individual is suffering from a respiratory-associated disease or condition, optionally wherein the respiratory-associated disease or condition includes pneumonia, inflammation, allergies, lung cancer, a bacterial infection, a viral infection, a cold, asthma, chronic obstructive pulmonary disease (COPD), cystic fibrosis, congestive heart failure, or any combination thereof.

8. The system (100) of any one of claims 1 to 7, wherein:
the respiratory therapy device (122) includes a motor and the acoustic signal is generated by operation of the motor; and/or
the system (100) further comprises a microphone (140) configured to detect the acoustic reflection of the acoustic signal and generate the sound signal; and/or
the system (100) further comprises a speaker (142) configured to generate the acoustic signal, optionally wherein the user interface (124) is configured to cover a mouth and a nose of the individual, and wherein the speaker (142) is at least partially positioned within the user interface (124).

9. The system (100) of any one of claims 1 to 8, wherein the one or more processors (122) of the control system (110) are further configured to execute the machine-readable instructions to modulate a frequency of the acoustic signal, an amplitude of the acoustic signal, or both, optionally wherein the modulation of the frequency, the amplitude, or both is in a continuous fashion or a step-wise fashion, and/or optionally wherein the frequency is between about 0 and about 5 Hz, between about 0 Hz and about 2 Hz, or about 2 Hz.

10. The system (100) of any one of claims 1 to 9, wherein:
the additional data includes personal data associated with the individual, optionally wherein the personal data includes an age of the individual, a sex of the individual, a gender of the individual, an ethnicity of the individual, a location of the individual, a known medical condition of the individual, a height of the individual, a weight of the individual, a neck circumference of the individual, or any combination thereof; and/or the additional data includes data generated by one or more sensors, data generated by one or more medical measurement devices, data generated by one or more smart devices, or any combination thereof, optionally wherein the additional data includes breathing rate data, heart rate data, pulse oximetry data, or any combination thereof; and/or
wherein the additional data is received from an electronic medical record of the individual, a smart device, or both.

11. The system (100) of any one of claims 1 to 10, wherein the one or more processors (122) of the control system (110) are further configured to execute the machine-readable instructions to:
instruct the individual to move to a specified physical position; and
in response to determining that the individual has moved to the specified physical position, direct the acoustic signal into the airway of the individual,
optionally wherein the specified physical position is a standing position, a seated position, an inclined position, or a laying position.

12. The system (100) of any one of claims 1 to 11, wherein the one or more processors (122) of the control system (110) are further configured to execute the machine-readable instructions to:
instruct the individual to breathe according to a specified breathing pattern; and
in response to determining that the individual is breathing according to the specified breathing pattern, direct the acoustic signal into the airway of the individual,
optionally wherein the specified breathing pattern includes the individual breathing heavily, the individual holding their breath, or both.

13. The system (100) of any one of claims 1 to 12, wherein the one or more processors (112) of the control system (110) are further configured to execute the machine-readable instructions to:
based on the determined value of the parameter in conjunction with the additional data, cause the action to be performed.

14. The system (100) of any one of claims 1 to 13, wherein the one or more processors (112) of the control system (110) are further configured to execute the machine-readable instructions to:
instruct the individual to move from a first physical position to a second physical position;
in response to determining that the individual has moved from the first physical position to the second physical position, direct the acoustic signal into the airway of the individual; and
analyze the acoustic data to determine the value of the parameter when the individual is in the second physical position, optionally wherein the determined value of the parameter is more accurate when the individual is in the second physical position than the first physical position.

15. The system (100) of any one of claims 1 to 14, wherein the one or more processors (112) of the control system (100) are further configured to execute the machine-readable instructions to:
instruct the individual to stop breathing according to a first breathing pattern and begin breathing according to a second breathing pattern;
in response to determining that the individual has begun to breath according to the second breathing pattern, direct the acoustic signal into the airway of the individual; and
analyze the acoustic data to determine the value of the parameter when the individual is breathing according to the second breathing pattern, optionally wherein the determined value of the parameter is more accurate when the individual is breathing according to the second breathing pattern than the first breathing pattern.

## Patentansprüche

1. System (100) zur Überwachung der Atemwege einer Person, wobei das System Folgendes umfasst:
ein Beatmungstherapiesystem (120), beinhaltend:
eine Beatmungstherapievorrichtung (122), die zum Zuleiten von Druckluft konfiguriert ist; und
eine Benutzerschnittstelle (124), die über eine Leitung (126) mit der Beatmungstherapievorrichtung (122) verbunden ist, wobei die Benutzerschnittstelle (124) so konfiguriert ist, dass sie mit der Person interagiert und dabei hilft, die zugeführte Druckluft in die Atemwege der Person zu lenken;
einen Speicher (114), der maschinenlesbare Anweisungen speichert; und
ein Steuerungssystem (110), das einen oder mehrere Prozessoren (112) beinhaltet, die so konfiguriert sind, dass sie die maschinenlesbaren Anweisungen ausführen, um:
zu bewirken, dass ein akustisches Signal über die Leitung (126) und die Benutzerschnittstelle (124) in die Atemwege der Person gelenkt wird;
akustische Daten zu erzeugen, die eine oder mehrere Reflexionen des akustischen Signals darstellen, die durch (i) einen Teil der Atemwege der Person, (ii) eine Verstopfung in den Atemwegen der Person oder (iii) sowohl (i) als auch (ii) verursacht werden;
die akustischen Daten zu analysieren, um einen Wert eines Parameters zu ermitteln, der mit den Atemwegen der Person zusammenhängt;
wobei der eine oder die mehreren Prozessoren (112) des Steuerungssystems (110) ferner so konfiguriert sind, dass sie die maschinenlesbaren Anweisungen ausführen, um: zusätzliche Daten zu empfangen, die mit der Person in Verbindung stehen;
den Wert des Parameters anhand der zusätzlichen Daten zu ändern; und
basierend auf dem geänderten Parameterwert und den zusätzlichen Daten zu veranlassen, dass eine Maßnahme durchgeführt wird.

2. System (100) nach Anspruch 1, wobei der Parameter eine Impedanz der Atemwege der Person ist.

3. System (100) nach Anspruch 1, wobei der Parameter eine Impedanz der Atemwege der Person, eine Resonanzfrequenz der Atemwege der Person, eine Lage der physischen Obstruktion, eine Größe der physischen Obstruktion oder eine beliebige Kombination davon ist.

4. System (100) nach einem der Ansprüche 1 bis 3, wobei die Maßnahme Veranlassen, dass ein Medikament in die Atemwege injiziert wird, Veranlassen, dass eine Benachrichtigung oder ein Bericht an die betroffene Person oder einen Dritten übermittelt oder dieser/diesem angezeigt wird, Generieren einer Therapieempfehlung, Anpassen eines Drucks, einer Durchflussrate oder beider der den Atemwegen der betroffenen Person zugeleiteten Druckluft oder eine beliebige Kombination davon beinhaltet; und/oder
die Druckluft mit einem ersten Druck in die Atemwege der Person geleitet wird, und die Maßnahme beinhaltet, dass die Beatmungstherapievorrichtung (122) die Druckluft mit einem zweiten Druck in die Atemwege des Patienten leitet, der größer als der erste Druck ist.

5. System (100) nach einem der Ansprüche 1 bis 4, wobei das Beatmungstherapiesystem (120) anfänglich als Atemwegüberdrucksystem arbeitet und wobei die Maßnahme Einstellen des Beatmungstherapiesystems (120) beinhaltet, so dass das Beatmungstherapiesystem (120) als Sauerstoffkonzentrator, Beatmungsgerät oder beides arbeitet.

6. System (100) nach einem der Ansprüche 1 bis 5, wobei der eine oder die mehreren Prozessoren (112) des Steuerungssystems (110) ferner so konfiguriert sind, dass sie die maschinenlesbaren Anweisungen ausführen, um den ermittelten Wert des Parameters mit einem zuvor erhaltenen Basiswert des Parameters zu vergleichen, und wobei optional:
die Maßnahme auf dem Vergleich zwischen dem ermittelten Wert des Parameters und dem zuvor ermittelten Basiswert des Parameters basiert und/oder der Basiswert des Parameters zuvor von der Person zu einem ersten Zeitpunkt erhalten wird, wobei das Steuerungssystem so konfiguriert ist, dass es die maschinenlesbaren Anweisungen ausführt, um den Wert des Parameters zu einem zweiten Zeitpunkt nach dem ersten Zeitpunkt zu ermitteln, wobei ein Zeitunterschied optional zwischen dem ersten und dem zweiten Zeitpunkt einen Tag, eine Woche, einen Monat oder ein Jahr beträgt, oder wobei der erste Zeitpunkt während einer ersten Jahreszeit und der zweite Zeitpunkt während einer zweiten Jahreszeit liegt; und/oder
der Basiswert des Parameters zuvor von einer anderen Person erhalten wird, die ein oder mehrere Merkmale mit der betreffenden Person teilt, wobei das eine oder die mehreren Merkmale optional Alter, Geschlecht, ethnische Zugehörigkeit, Wohnort, medizinische Zustände, Körpergröße, Gewicht, Halsumfang oder eine beliebige Kombination davon beinhalten; und/oder
der Basiswert des Parameters zuvor von einer anderen Person mit einer Diagnose einer Atemwegserkrankung oder eines Atemwegszustands erhalten wird und wobei der Vergleich zwischen dem ermittelten Wert des Parameters und dem Basiswert des Parameters angibt, ob die Person an der Atemwegserkrankung oder dem Atemwegszustand leidet.

7. System (100) nach einem der Ansprüche 1 bis 6, wobei der Wert des Parameters angibt, ob die Person an einer Atemwegserkrankung oder einem Atemwegszustand leidet, wobei die Atemwegserkrankung oder der Atemwegszustand optional Pneumonie, Entzündungen, Allergien, Lungenkrebs, eine bakterielle Infektion, eine Virusinfektion, eine Erkältung, Asthma, chronisch obstruktive Lungenerkrankung (COPD), Mukoviszidose, Herzinsuffizienz oder eine Kombination davon beinhaltet.

8. System (100) nach einem der Ansprüche 1 bis 7, wobei:
die Beatmungstherapievorrichtung (122) einen Motor beinhaltet und das akustische Signal durch Betrieb des Motors erzeugt wird und/oder
das System (100) ferner ein Mikrofon (140) umfasst, das so konfiguriert ist, dass es die akustische Reflexion des akustischen Signals erkennt und das Schallsignal erzeugt; und/oder
das System (100) ferner einen Lautsprecher (142) umfasst, der so konfiguriert ist, dass er das akustische Signal erzeugt, wobei die Benutzerschnittstelle (124) optional so konfiguriert ist, dass sie Mund und Nase der Person bedeckt, und wobei der Lautsprecher (142) zumindest teilweise innerhalb der Benutzerschnittstelle (124) positioniert ist.

9. System (100) nach einem der Ansprüche 1 bis 8, wobei der eine oder die mehreren Prozessoren (122) des Steuerungssystems (110) ferner so konfiguriert sind, dass sie die maschinenlesbaren Anweisungen ausführen, um eine Frequenz des akustischen Signals, eine Amplitude des akustischen Signals oder beide zu modulieren, wobei die Modulation der Frequenz, der Amplitude oder beider optional kontinuierlich oder schrittweise erfolgt und/oder wobei die Frequenz optional zwischen etwa 0 und etwa 5 Hz, zwischen etwa 0 Hz und etwa 2 Hz oder bei etwa 2 Hz liegt.

10. System (100) nach einem der Ansprüche 1 bis 9, wobei:
die zusätzlichen Daten personenbezogene Daten, die mit der Person in Verbindung stehen, beinhalten, wobei die personenbezogenen Daten optional Alter der Person, Geschlecht der Person, Geschlechtsidentität der Person, ethnische Zugehörigkeit der Person, Aufenthaltsort der Person, einen bekannten medizinischen Zustand der Person, Körpergröße der Person, Gewicht der Person, Halsumfang der Person oder eine Kombination davon beinhalten; und/oder die zusätzlichen Daten Daten, die von einem oder mehreren Sensoren erzeugt werden, Daten, die von einer oder mehreren medizinischen Messvorrichtungen erzeugt werden, Daten, die von einer oder mehreren intelligenten Vorrichtungen erzeugt werden, oder eine beliebige Kombination davon beinhalten, wobei die zusätzlichen Daten optional Daten zur Atemfrequenz, zur Herzfrequenz, zur Pulsoximetrie oder eine beliebige Kombination davon beinhalten; und/oder
wobei die zusätzlichen Daten aus einer elektronischen Patientenakte der betreffenden Person, einer intelligenten Vorrichtung oder beiden stammen.

11. System (100) nach einem der Ansprüche 1 bis 10, wobei der eine oder die mehreren Prozessoren (122) des Steuerungssystems (110) ferner so konfiguriert sind, dass sie die maschinenlesbaren Anweisungen ausführen, um:
die betreffende Person anzuweisen, eine bestimmte Körperhaltung einzunehmen; und
sobald festgestellt wird, dass die Person bestimmte Körperhaltung eingenommen hat, das akustische Signal in die Atemwege der Person zu lenken,
wobei die bestimmte Körperhaltung optional eine stehende Position, eine sitzende Position, eine geneigte Position oder eine liegende Position ist.

12. System (100) nach einem der Ansprüche 1 bis 11, wobei der eine oder die mehreren Prozessoren (122) des Steuerungssystems (110) ferner so konfiguriert sind, dass sie die maschinenlesbaren Anweisungen ausführen, um:
die betreffende Person anzuweisen, nach einem bestimmten Atemmuster zu atmen; und
sobald festgestellt wird, dass die Person nach dem bestimmten Atemmuster atmet, das akustische Signal in die Atemwege der Person zu lenken,
wobei das angegebene Atemmuster optional tiefes Atmen der Person, Anhalten des Atems oder beides beinhaltet.

13. System (100) nach einem der Ansprüche 1 bis 12, wobei der eine oder die mehreren Prozessoren (112) des Steuerungssystems (110) ferner so konfiguriert sind, dass sie die maschinenlesbaren Anweisungen ausführen, um:
basierend auf dem ermittelten Parameterwert in Verbindung mit den zusätzlichen Daten Durchführen der entsprechenden Maßnahme zu veranlassen.

14. System (100) nach einem der Ansprüche 1 bis 13, wobei der eine oder die mehreren Prozessoren (112) des Steuerungssystems (110) ferner so konfiguriert sind, dass sie die maschinenlesbaren Anweisungen ausführen, um:
die Person anzuweisen, von einer ersten Körperhaltung in eine zweite Körperhaltung zu wechseln;
sobald festgestellt wird, dass die Person von der ersten Körperhaltung in die zweite Körperhaltung gewechselt hat, das akustische Signal in die Atemwege der Person zu lenken; und
die akustischen Daten zu analysieren, um den Wert des Parameters zu ermitteln, wenn die Person die zweite Körperhaltung eingenommen hat, wobei der ermittelte Wert des Parameters in der zweiten Körperhaltung optional genauer ist als in der ersten Körperhaltung.

15. System (100) nach einem der Ansprüche 1 bis 14, wobei der eine oder die mehreren Prozessoren (112) des Steuerungssystems (100) ferner so konfiguriert sind, dass sie die maschinenlesbaren Anweisungen ausführen, um:
die Person anzuweisen, Atmen gemäß einem ersten Atemmuster zu stoppen und Atmen gemäß einem zweiten Atemmuster zu beginnen;
sobald festgestellt wird, dass die Person gemäß dem zweiten Atemmuster zu atmen begonnen hat, das akustische Signal in die Atemwege der Person zu lenken; und
die akustischen Daten zu analysieren, um den Wert des Parameters zu ermitteln, wenn die Person nach dem zweiten Atemmuster atmet, wobei der ermittelte Wert des Parameters optional genauer ist, wenn die Person nach dem zweiten Atemmuster atmet als nach dem ersten Atemmuster.

## Revendications

1. Système (100) de surveillance des voies respiratoires d'un individu, le système comprenant :
un système de thérapie respiratoire (120) incluant :
un dispositif de thérapie respiratoire (122) configuré pour fournir de l'air sous pression ; et
une interface utilisateur (124) couplée au dispositif de thérapie respiratoire (122) via un conduit (126), l'interface utilisateur (124) étant configurée pour engager l'individu et aider à diriger l'air sous pression fourni vers les voies respiratoires de l'individu ;
une mémoire (114) stockant des instructions lisibles par machine ; et
un système de commande (110) incluant un ou plusieurs processeurs (112) configurés pour exécuter les instructions lisibles par machine pour :
amener un signal acoustique à être dirigé, via le conduit (126) et l'interface utilisateur (124), dans les voies respiratoires de l'individu ;
générer des données acoustiques représentatives d'une ou plusieurs réflexions du signal acoustique causées par (i) une partie des voies respiratoires de l'individu, (ii) une obstruction dans les voies respiratoires de l'individu, ou (iii) à la fois (i) et (ii) ;
analyser les données acoustiques pour déterminer une valeur d'un paramètre associé aux voies respiratoires de l'individu ;
dans lequel les un ou plusieurs processeurs (112) du système de commande (110) sont en outre configurés pour exécuter les instructions lisibles par machine pour : recevoir des données supplémentaires associées à l'individu ;
modifier la valeur du paramètre sur la base des données supplémentaires ; et
sur la base de la valeur modifiée du paramètre et des données supplémentaires, amener une action à être exécutée.

2. Système (100) selon la revendication 1, dans lequel le paramètre est une impédance des voies respiratoires de l'individu.

3. Système (100) selon la revendication 1, dans lequel le paramètre est une impédance des voies respiratoires de l'individu, une fréquence de résonance des voies respiratoires de l'individu, un emplacement de l'obstruction physique, une taille de l'obstruction physique ou toute combinaison de ceux-ci.

4. Système (100) selon l'une quelconque des revendications 1 à 3, dans lequel l'action inclut le fait d'amener un médicament à être injecté dans les voies respiratoires, le fait d'amener une notification ou un rapport à être transmis ou affiché à l'individu ou à un tiers, la génération d'une recommandation de thérapie, l'ajustement d'une pression, d'un débit ou des deux, de l'air sous pression fourni aux voies respiratoires de l'individu, ou toute combinaison de ceux-ci ; et/ou
l'air sous pression est fourni aux voies respiratoires de l'individu à une première pression, et l'action inclut le fait d'amener le dispositif de thérapie respiratoire (122) à fournir l'air sous pression aux voies respiratoires de l'individu à une seconde pression qui est supérieure à la première pression.

5. Système (100) selon l'une quelconque des revendications 1 à 4, dans lequel le système de thérapie respiratoire (120) fonctionne initialement comme un système de pression positive des voies respiratoires, et dans lequel l'action inclut l'ajustement du système de thérapie respiratoire (120) de sorte que le système de thérapie respiratoire (120) fonctionne comme un concentrateur d'oxygène, un ventilateur ou les deux.

6. Système (100) selon l'une quelconque des revendications 1 à 5, dans lequel les un ou plusieurs processeurs (112) du système de commande (110) sont en outre configurés pour exécuter les instructions lisibles par machine afin de comparer la valeur déterminée du paramètre avec une valeur de référence précédemment obtenue du paramètre, et, facultativement dans lequel :
l'action est basée sur la comparaison entre la valeur déterminée du paramètre et la valeur de référence précédemment déterminée du paramètre, et/ou la valeur de référence du paramètre est précédemment obtenue auprès de l'individu à un premier moment, et dans lequel le système de commande est configuré pour exécuter les instructions lisibles par machine afin de déterminer la valeur du paramètre à un second moment qui se situe après le premier moment, facultativement dans lequel une différence de temps entre le premier moment et le second moment est un jour, une semaine, un mois ou un an, ou dans lequel le premier moment se situe au cours d'une première saison de l'année, et dans lequel le second moment se situe au cours d'une seconde saison de l'année ; et/ou
la valeur de référence du paramètre est précédemment obtenue auprès d'un autre individu partageant une ou plusieurs caractéristiques avec l'individu, facultativement dans lequel les une ou plusieurs caractéristiques incluent l'âge, le sexe, le genre, l'origine ethnique, le lieu de résidence, les conditions médicales, la taille, le poids, le tour de cou ou toute combinaison de ceux-ci ; et/ou
la valeur de référence du paramètre est précédemment obtenue auprès d'un autre individu chez qui une maladie ou un trouble respiratoire a été diagnostiqué, et dans lequel la comparaison entre la valeur déterminée du paramètre et la valeur de référence du paramètre indique si l'individu est atteint de la maladie ou du trouble respiratoire.

7. Système (100) selon l'une quelconque des revendications 1 à 6, dans lequel la valeur du paramètre indique si l'individu souffre d'une maladie ou d'un trouble respiratoire, facultativement dans lequel la maladie ou le trouble respiratoire inclut une pneumonie, une inflammation, des allergies, un cancer du poumon, une infection bactérienne, une infection virale, un rhume, l'asthme, une bronchopneumopathie chronique obstructive (BPCO), la fibrose kystique, une insuffisance cardiaque congestive ou toute combinaison de celles-ci.

8. Système (100) selon l'une quelconque des revendications 1 à 7, dans lequel :
le dispositif de thérapie respiratoire (122) inclut un moteur et le signal acoustique est généré par le fonctionnement du moteur ; et/ou
le système (100) comprend en outre un microphone (140) configuré pour détecter la réflexion acoustique du signal acoustique et générer le signal sonore ; et/ou
le système (100) comprend en outre un haut-parleur (142) configuré pour générer le signal acoustique, facultativement dans lequel l'interface utilisateur (124) est configurée pour couvrir la bouche et le nez de l'individu, et dans lequel le haut-parleur (142) est au moins partiellement positionné à l'intérieur de l'interface utilisateur (124).

9. Système (100) selon l'une quelconque des revendications 1 à 8, dans lequel les un ou plusieurs processeurs (122) du système de commande (110) sont en outre configurés pour exécuter les instructions lisibles par machine afin de moduler une fréquence du signal acoustique, une amplitude du signal acoustique, ou les deux, facultativement dans lequel la modulation de la fréquence, de l'amplitude, ou des deux est continue ou par paliers, et/ou facultativement dans lequel la fréquence est comprise entre environ 0 et environ 5 Hz, entre environ 0 Hz et environ 2 Hz, ou autour de 2 Hz.

10. Système (100) selon l'une quelconque des revendications 1 à 9, dans lequel :
les données supplémentaires incluent des données personnelles associées à l'individu, facultativement dans lequel les données personnelles incluent l'âge de l'individu, le sexe de l'individu, le genre de l'individu, l'origine ethnique de l'individu, le lieu de résidence de l'individu, un problème médical connu de l'individu, la taille de l'individu, le poids de l'individu, le tour de cou de l'individu, ou toute combinaison de ceux-ci ; et/ou les données supplémentaires incluent des données générées par un ou plusieurs capteurs, des données générées par un ou plusieurs dispositifs de mesure médicale, des données générées par un ou plusieurs dispositifs intelligents, ou toute combinaison de ceux-ci, facultativement dans lequel les données supplémentaires incluent des données sur la fréquence respiratoire, des données sur la fréquence cardiaque, des données d'oxymétrie de pouls, ou toute combinaison de ceux-ci ; et/ou
dans lequel les données supplémentaires sont reçues à partir d'un dossier médical électronique de l'individu, d'un dispositif intelligent, ou des deux.

11. Système (100) selon l'une quelconque des revendications 1 à 10, dans lequel les un ou plusieurs processeurs (122) du système de commande (110) sont en outre configurés pour exécuter les instructions lisibles par machine pour :
ordonner à l'individu de se déplacer vers une position physique spécifiée ; et
en réponse à la détermination du fait que l'individu s'est déplacé vers la position physique spécifiée, diriger le signal acoustique dans les voies respiratoires de l'individu,
facultativement dans lequel la position physique spécifiée est une position debout, une position assise, une position inclinée ou une position couchée.

12. Système (100) selon l'une quelconque des revendications 1 à 11, dans lequel les un ou plusieurs processeurs (122) du système de commande (110) sont en outre configurés pour exécuter les instructions lisibles par machine pour :
ordonner à l'individu de respirer selon un schéma respiratoire spécifié ; et
en réponse à la détermination du fait que l'individu respire selon le schéma respiratoire spécifié, diriger le signal acoustique dans les voies respiratoires de l'individu,
facultativement dans lequel le schéma respiratoire spécifié inclut le fait que l'individu respire fortement, que l'individu retient son souffle, ou les deux.

13. Système (100) selon l'une quelconque des revendications 1 à 12, dans lequel les un ou plusieurs processeurs (112) du système de commande (110) sont en outre configurés pour exécuter les instructions lisibles par machine pour :
sur la base de la valeur déterminée du paramètre et des données supplémentaires, amener l'action à être exécutée.

14. Système (100) selon l'une quelconque des revendications 1 à 13, dans lequel les un ou plusieurs processeurs (112) du système de commande (110) sont en outre configurés pour exécuter les instructions lisibles par machine pour :
ordonner à l'individu de se déplacer d'une première position physique à une seconde position physique ;
en réponse à la détermination du fait que l'individu s'est déplacé de la première position physique à la seconde position physique, diriger le signal acoustique dans les voies respiratoires de l'individu ; et
analyser les données acoustiques pour déterminer la valeur du paramètre lorsque l'individu se trouve dans la seconde position physique, facultativement dans lequel la valeur déterminée du paramètre est plus précise lorsque l'individu se trouve dans la seconde position physique que dans la première position physique.

15. Système (100) selon l'une quelconque des revendications 1 à 14, dans lequel les un ou plusieurs processeurs (112) du système de commande (100) sont en outre configurés pour exécuter les instructions lisibles par machine pour :
ordonner à l'individu d'arrêter de respirer selon un premier schéma respiratoire et de commencer à respirer selon un second schéma respiratoire ;
en réponse à la détermination du fait que l'individu a commencé à respirer selon le second schéma respiratoire, diriger le signal acoustique dans les voies respiratoires de l'individu ; et
analyser les données acoustiques pour déterminer la valeur du paramètre lorsque l'individu respire selon le second schéma respiratoire, facultativement dans lequel la valeur déterminée du paramètre est plus précise lorsque l'individu respire selon le second schéma respiratoire que selon le premier schéma respiratoire.
